(19) Europäisches Patentamt
European Patent Office
Office européen des brevets

(11) Veröffentlichungsnummer: **0 122 882**
**A1**

(12) # EUROPÄISCHE PATENTANMELDUNG

(21) Anmeldenummer: **84810178.8**

(22) Anmeldetag: **10.04.84**

(51) Int. Cl.³: **C 07 J 71/00**
A 61 K 31/58, A 61 K 31/585
//C07J21/00

(30) Priorität: **13.04.83 CH 1979/83**

(43) Veröffentlichungstag der Anmeldung:
**24.10.84 Patentblatt 84/43**

(84) Benannte Vertragsstaaten:
**AT BE CH DE FR GB IT LI LU NL SE**

(71) Anmelder: **CIBA-GEIGY AG**
**Postfach**
**CH-4002 Basel(CH)**

(72) Erfinder: **Grob, Jürgen**
**Hauptstrasse 25**
**CH-4304 Giebenach(CH)**

(72) Erfinder: **Kalvoda, Jaroslav, Dr.**
**Leimgrubenweg 21**
**CH-4102 Binningen(CH)**

(54) **Neue Steroide der 20-Spiroxanreihe, Verfahren zu ihrer Herstellung, pharmazeutische Präparate enthaltend solche Verbindungen und Verwendung der letzteren.**

(57) (5α)-20-Spirox-1-en-3-one (I) und 17-Hydroxy-5α, 17α-pregn-1-en-3-one (II) der Formeln

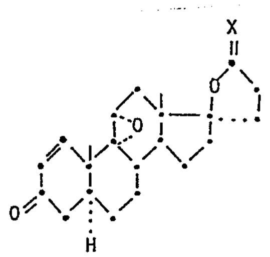

(I)

bzw.

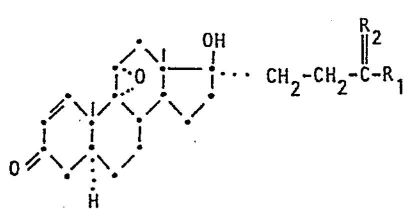

(II),

worin X = O oder $H_2$, $R_2$ = O und $R_1$ = OH, OMe, OAlk, $NH_2$, $NHR_3$, $NR_3R_4$ oder $R_2$ = $H_2$ und $R_1$ = OH, OAlk, OAr, OAralk oder OAc ist.

wobei $R_3$, $R_4$ und Alk nieder-Alkyl, Me ein Metallatom bzw. -äquivalent oder das Kation einer organischen Base, Ar monocyclisches Aryl, Aralk monocyclisches Aryl-nieder-Alkyl, und Ac nieder-Alkanoyl, monocyclisches Aroyl, nieder-Alkylsulfonyl oder monocyclisches Arylsulfonyl bedeuten, wobei $R_3$ mit $R_4$ zusammen auch eine gegebenenfalls durch ein Heteroatom unterbrochene nieder-Alkylengruppe bedeuten kann, werden durch an sich bekannte Analogieverfahren hergestellt. Sie zeigen eine Aldosteron-antagonisierende Wirkung und können daher z.B. als Kalium-sparende Diuretika, gegebenenfalls auch zusammen mit einem anderen, gegen Elektrolyte nicht spezifischen Diuretikum oder Saluretikum verwendet werden.

EP 0 122 882 A1

Croydon Printing Company Ltd.

CIBA-GEIGY AG
Basel (Schweiz)

4-14386/=

Neue Steroide der 20-Spiroxanreihe, Verfahren zu ihrer Herstellung, pharmazeutische Präparate enthaltend solche Verbindungen und Verwendung der letzteren.

Die Erfindung betrifft neue (5α)-20-Spirox-1-en-3-one der Formel

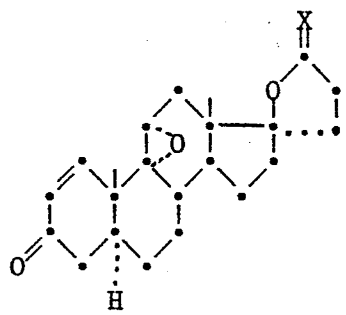

(I)

und sich davon ableitende Derivate mit dem geöffneten spiro-verknüpften Ring in 17-Stellung, nämlich 17-Hydroxy-5α,17α-pregn-1-en-3-one der Formel

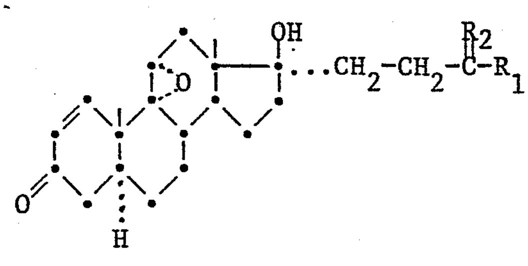

(II),

worin X = O oder $H_2$, $R_2$ = O und $R_1$ = OH, OMe, OAlk, $NH_2$, $NHR_3$, $NR_3R_4$
oder $R_2$ = $H_2$ und $R_1$ = OH, OAlk, OAr, OAralk oder OAc
ist,

wobei $R_3$, $R_4$ und Alk nieder-Alkyl, Me ein Metallatom bzw. -äquivalent oder das Kation einer organischen Base, Ar monocyclisches Aryl, Aralk monocyclisches Aryl-nieder-Alkyl, und Ac nieder-Alkanoyl,

monocyclisches Aroyl, nieder-Alkylsulfonyl oder monocyclisches Arylsulfonyl

bedeuten, wobei $R_3$ mit $R_4$ zusammen auch eine gegebenenfalls durch ein Heteroatom unterbrochene nieder-Alkylengruppe bedeuten kann, Verfahren zu ihrer Herstellung, pharmazeutische Präparate mit einem Gehalt an diesen Verbindungen, die therapeutische Verwendung der Verbindungen als Kalium-sparende Diuretika, sowie Zusammensetzungen der neuen Verbindungen mit Diuretika oder Saluretika. Die Erfindung betrifft sodann auch die Verwendung der neuen Verbindungen bei Therapien, die von Diuretika oder Saluretika Gebrauch machen, bei denen sie gleichzeitig oder nacheinander mit diesen Medikamenten verabreicht werden.

Unter "20-Spiroxan" wird das folgende Ringsystem

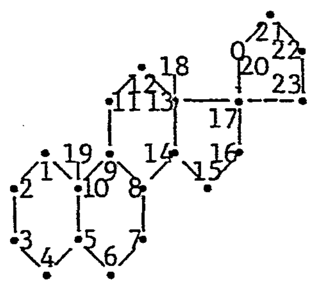

verstanden, vgl. z.B. UK Patentschrift 1.041.534. Das den obigen Verbindungen der Formel (I) zugrunde liegende Gerüst wird hier als "(5α)-20-Spiroxan" bezeichnet. Die Verbindung gemäss Formel (I), in welcher X = $H_2$ ist, stellt somit das 9α,11α-Epoxy-(5α)-20-spirox-1-en-3-on und die entsprechende Verbindung, worin X = O ist, das 9α,11α-Epoxy-(5α)-20-spirox-1-en-3,21-dion dar.

Verbindungen der Formel (II), in denen $R_2$ = O und $R_1$ eine freie oder funktionell abgewandelte Hydroxy- oder Aminogruppe ist, stellen die 9α,11α-Epoxy-17—hydroxy-3-oxo—5α,17α-pregn-1-en-21-carbonsäure, ihre Metallsalze oder Salze mit organischen Basen, ihre Ester, Amide oder substituierten Amide dar. Die Verbindung der Formel (I), in der X = O, ist das γ-Lacton der Carbonsäure gemäss Formel (II).

Verbindung der Formel (II), in welcher $R_2 = H_2$ und $R_1$ eine freie oder funktionell abgewandelte Hydroxygruppe bedeutet, stellt das 9α,11α-Epoxy-17β-hydroxy-17α-(3-hydroxypropyl)—5α—androst-1-en-3-on oder seine Ester und Aether dar. Das Cyclisierungsprodukt des freien Alkohols ist die Verbindung der Formel (I), worin $X = H_2$ ist, d.h. das 9α,11α-Epoxy-(5α)-20-spirox-1-en-3-on.

Der Begriff "nieder" als auf Alkyl- oder Alkanoylgruppen bezogen, bedeutet einen solchen Rest mit 1-7 C-Atomen, z.B. Methyl, Aethyl, n-Propyl, iso-Propyl, n-Butyl, iso-Butyl, tert.-Butyl, n-Pentyl, n-Hexyl bzw. Formyl, Acetyl, Propionyl, Butyryl, Valeroyl. Monocyclisches Aryl ist insbesondere Phenyl oder dessen Substitutionsderivate mit 1-3 Niederalkylgruppen, wie insbesondere Methylgruppen, und/oder Halogenatomen, wie insbesondere Chlor- oder Bromatomen. Monocyclisches Aryl-niederalkyl ist z.B. Phenylmethyl oder Phenyläthyl. Monocyclisches Aroyl ist z.B. Benzoyl oder ein sich davon durch Substitution mit Hydroxy, Amino oder Methyl ergebendes Radikal, wie z.B. Salicyloyl oder Anthranoyl. Monocyclisches Arylsulfonyl ist z.B. p-Toluolsulfonyl oder Benzolsulfonyl, Niederalkylsulfonyl ist z.B. Methyl- oder Aethylsulfonyl. Eine niedere Alkylengruppe weist vorzugsweise 2-6 C-Atome auf, vorzugsweise ist sie eine Alkylengruppe mit gerader Kohlenstoffkette. Aether gemäss der obigen Formel leiten sich daher von niederen aliphatischen Alkoholen oder gegebenenfalls von monocyclischen aromatischen Alkoholen oder von monocyclischen araliphatischen Alkoholen ab. Die substituierten Amide enthalten einen oder zwei gleiche oder verschiedene nieder-Alkylreste oder sind ringförmig gebaut und leiten sich im letzteren Falle z.B. von Pyrrolidin oder Piperidin ab. Da die Alkylengruppe $-R_3R_4-$ auch durch ein Heteroatom, wie insbesondere Stickstoff, Sauerstoff oder Schwefel unterbrochen sein kann, kommen auch Amide in Betracht, die sich z.B. von Piperazin oder Morpholin ableiten. Metallsalze der 9α,11α-Epoxy-17-hydroxy-3-oxo-5α,17α-pregn-1-en-21-carbonsäure sind insbesondere die Alkali- oder Erdalkalimetallsalze, wie das Natrium oder Kaliumsalz, ferner das Ammoniumsalz, das Magnesiumsalz oder das Calciumsalz.

Als Salze von organischen Basen sind insbesondere Ammoniumsalze von geeigneten, vorzugsweise physiologisch verträglichen, organischen stickstoffhaltigen Basen hervorzuheben. Als Basen kommen sowohl Amine, wie Niederalkylamine, z.B. Triäthylamin, Hydroxyniederalkylamine, z.B. 2-Hydroxyäthylamin, Di-(2-Hydroxyäthyl)-amin oder Tri-(2-hydroxyäthyl)-amin, Cycloalkylamine, z.B. Dicyclohexylamin, oder Benzylamine, z.B. Benzylamin und N,N'-Dibenzyläthylendiamin, als auch stickstoffhaltige heterocyclische Verbindungen, z.B. solche aromatischen Charakters, wie Pyridin oder Chinolin, oder solche mit einem mindestens teilweise gesättigten heterocyclischen Ring, wie N-Aethylpiperidin, Morpholin, Piperazin oder N,N'-Dimethylpiperazin, in Betracht.

Unter den spezifischen Verbindungen gemäss der Erfindung sind ausser den schon genannten
9α,11α-Epoxy-(5α)-20-spirox-1-en-3,21-dion
9α,11α-Epoxy-(5α)-20-spirox-1-en-3-on
9α,11α-Epoxy-17—hydroxy-3-oxo——5α,17α-pregn-1-en-21-carbonsäure
9α,11α-Epoxy-17—hydroxy-17α-(3-hydroxypropyl)—5α—androst-1-en-3-on,

ihre Aether und Ester zu erwähnen, die sich von Nieder-Alkanolen mit 1-4 C-Atomen, bzw. von Alkansäuren mit 1-4 C-Atomen ableiten, und insbesondere auch das Kalium-, Natrium und Ammoniumsalz der 9α,11α-Epoxy-17-hydroxy-3-oxo-5α,17α-pregn-1-en-21-carbonsäure, sowie ihr Methyl-, Aethyl- und Propylester und das unsubstituierte Amid und die Mono- oder di-Methyl-, -Aethyl- oder -Propylamide; sodann die Methyl-, Aethyl- und Propyl-Aether des 9α,11α-Epoxy-17-hydroxy-17α-(3-hydroxypropyl)-(5α)-androst-1-en-3-ons.

Die erfinderungsgemässen Verbindungen der Formeln I und II zeichnen sich durch günstige biologische Eigenschaften aus und stellen somit wertvolle pharmazeutische Wirkstoffe dar. Insbesondere weisen sie eine starke Aldosteron-antagonisierende Wirkung auf und können als Kalium-sparende Diuretika Anwendung in der Therapie finden. So zeigen sie im Kagawa-Test an adrenalektomierten, mit 1 µg Aldosteron behandelten

männlichen Ratten bei der peroralen Applikation eine antimineralocorticoide Wirkung schon in Dosen von ca. 0,5 mg/kg und besitzen eine $ED_{50}$ zwischen 1-10 mg/kg, bei einer Wirkungsdauer von ca. 3-7 Stunden. Das bekannte Spironolacton, das stärkste im Handel befindliche Antimineralocorticoid, besitzt im Vergleich dazu im gleichen Test unter denselben Bedingungen eine $ED_{50}$ von ca. 5 mg/kg. Die antimineralocorticoide Wirkung der neuen Verbindungen gemäss der Erfindung erscheint somit ausgeprägter als bei den bisher bekannten Medikamenten dieser Art. Besonders hervorzuheben ist die sehr gute Verträglichkeit der neuen Verbindungen. In dieser Beziehung sind sie z.B. dem oben genannten Spironolacton überlegen. So kann weder an intakten noch an kastrierten männlichen Ratten bei Dosen bis über 100 mg/kg eine antiandrogene Wirkung nachgewiesen werden. Auch bei Gaben von 3 mg/kg (300 Mal die aktive Dosis von Progesteron) kann im McPhail-Test an Kaninchen keine gestagene Wirkung festgestellt werden.

Die neuen Verbindungen können daher in der Therapie für all jene Indikationen Verwendung finden, bei denen eine Aldosteron-antagonisierende Wirkung erwünscht ist, z.B. als Kalium-sparende Diuretika und als Antihypertensiva. Sie sind insbesondere auch bei Leberzirrhose und Herzinsuffizienz verwendbar.

Die erfindungsgemässen Verbindungen der Formeln I und II können in an sich bekannter Weise hergestellt werden. Das Verfahren zu ihrer Herstellung gemäss vorliegender Erfindung ist dadurch gekennzeichnet, dass man

a) eine Verbindung mit einer der Formeln

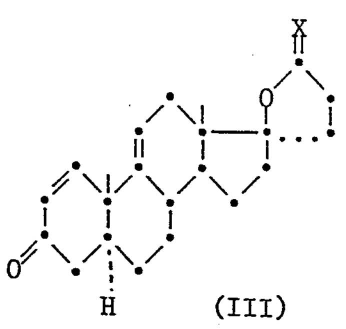

(III)

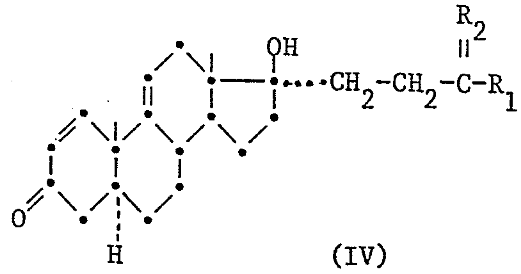

(IV)

in denen X, $R_1$ und $R_2$ die gleiche Bedeutung wie für Formeln (I) bzw. (II) haben, durch Behandlung mit einer Persäure zum entsprechenden 9α,11α-Epoxyd oxydiert, oder

b) in einer Verbindung mit einer der Formeln

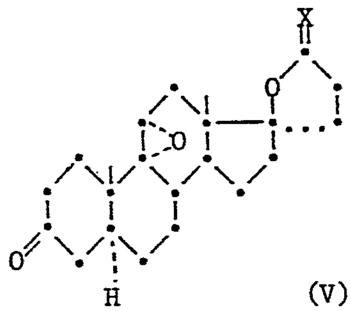

(V)

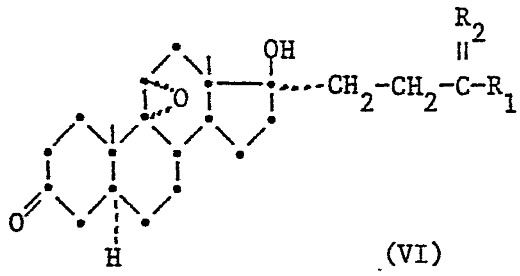

(VI)

in denen X, $R_1$ und $R_2$ die gleiche Bedeutung wie für Formeln (I) bzw. (II) haben, auf chemischem Wege eine Doppelbindung in 1,2-Stellung einführt, oder

c) in einer Verbindung mit einer der Formeln

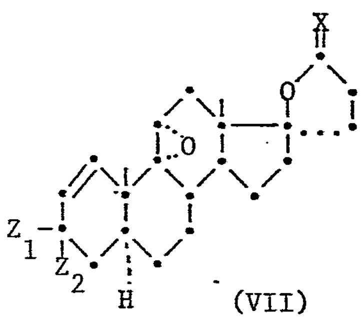

(VII)

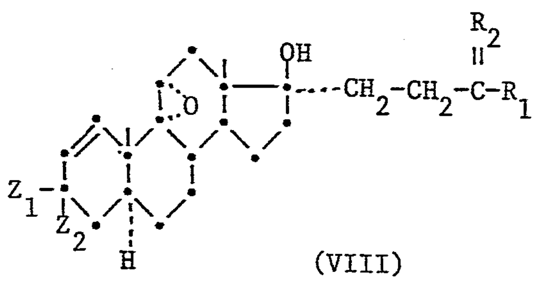

(VIII)

worin X, $R_1$ und $R_2$ die oben gegebenen Bedeutungen besitzen, und $Z_1$ und $Z_2$ eine in die Oxogruppe überführbare Gruppierung bedeutet, die Gruppe $Z_1$ und $Z_2$ in die 3-Oxogruppe überführt, oder

d) in einer Verbindung der Formel

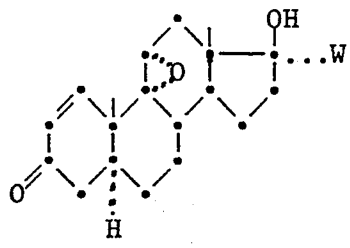

(IX),

worin W eine in 3'-Stellung durch eine reaktionsfähige funktionelle Gruppe oder eine sich davon ableitende abgewandelte Gruppe substituierte Propylgruppe, oder eine durch eine freie, veresterte oder amidierte Carboxylgruppe substituierte Aethylgruppe bedeutet, die Gruppe W unter Bildung eines Spiroäthers- oder Spirolactonringes mit der 17β-OH-Gruppe zur Reaktion bringt, oder

e) in einer Verbindung der Formel

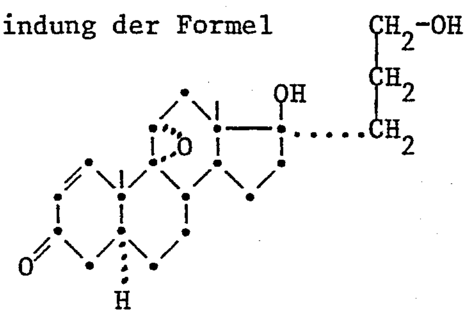

(X)

die Carbinolgruppe zur Carboxylgruppe gegebenenfalls unter Cyclisierung mit der 17β-Hydroxygruppe oxydiert, oder

f) : eine Verbindung der Formel

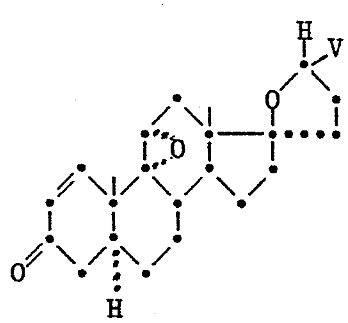

(XI)

in welcher V = H ist oder für eine freie, veresterte oder verätherte Hydroxy- oder Mercaptogruppe steht, zum entsprechenden Spirolacton oxydiert, oder

g) in einer Verbindung der Formel

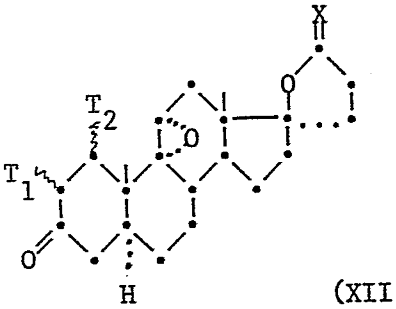

(XII)

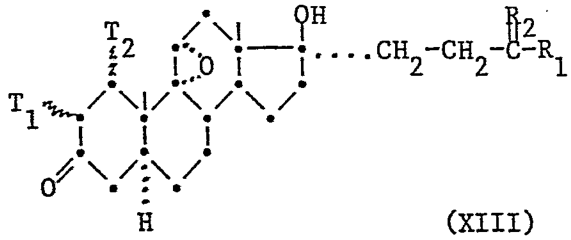

(XIII)

worin X, $R_1$ und $R_2$ die oben gegebene Bedeutung haben und eines der Substituenten $T_1$ und $T_2$ eine Abgangsgruppe ist und der andere Wasserstoff bedeutet, die Abgangsgruppe unter Bildung der 1,2-Doppelbindung abspaltet, oder

h) in einer Verbindung der Formel (II), worin in der 17α-Seitenkette eine freie Hydroxy- oder Carboxylgruppe vorhanden ist, diese funktionell abwandelt, oder in einer Verbindung der Formel (II), worin eine funktionell abgewandelte Hydroxy- oder Carboxylgruppe vorhanden ist, diese in die entsprechende freie Gruppe überführt, oder

i) eine Verbindung der Formel (I), worin X = O, in eine entsprechende Carbonsäure gemäss Formel (II) überführt,
und, wenn erwünscht, die nach irgend einem der vorhergehenden Verfahren erhaltene 9α,11α-Epoxy-17-hydroxy-3-oxo-5α,17α-pregn-1-en-21-carbonsäure in ihre Metallsalze oder Salze von organischen Basen überführt oder ein nach einem der genannten Verfahren erhaltenes Salz in die freie Säure überführt.

Die oben angegebenen Verfahren können in an sich bekannter Weise ausgeführt werden.

Die verfahrensgemässe Epoxidierung der 9,11-Doppelbindung in Verbindungen der Formeln (III) oder (IV) nach obiger Variante a), kann in an sich bekannter Weise ausgeführt werden. Man verwendet vorzugsweise

organische Persäuren, wie Perphthalsäure, Perbenzoesäure, Peressigsäure oder m-Chlorperbenzoesäure, und führt die Reaktion in einem inerten Lösungsmittel, wie z.B. einem Aether, z.B. Diäthyläther, oder einem aromatischen oder einem vorzugsweise halogenierten aliphatischen Kohlenwasserstoff bei niedriger Temperatur, z.B. bei 0 - 10° oder bei Raumtemperatur oder leicht erhöhter Temperatur aus.

Nach der obigen Variante b) des erfindungsgemässen Verfahrens führt man in die 1,2-Stellung der Ausgangsstoffe der Formeln V oder VI, z.B. nach an sich bekannten chemischen Methoden, eine Doppelbindung ein.

So kann man die Doppelbindung z.B. dadurch einführen, dass man die Ausgangsstoffe mit einem dehydrierend wirkenden Chinon bei Temperaturen zwischen ca. -5° und ca. 150° behandelt. Diese Dehydrierung kann in an sich bekannter Weise ausgeführt werden.

Man verwendet vorzugsweise 2,3-Dichlor-5,6-dicyan-1,4-benzochinon und arbeitet vorzugsweise bei Siedehitze in organischen Lösungsmitteln, z.B. aromatischen Kohlenwasserstoffen, wie Benzol, Toluol oder Xylol, niederaliphatischen Alkoholen, wie Aethanol, Propanol oder tert. Butylalkohol, niederaliphatischen Estern, wie Aethylacetat, insbesondere aber in cyclischen Aethern, wie Dioxan oder Tetrahydrofuran.

Man kann die Dehydrierung auch an einem $\Delta^2$-3-Enoläther als Ausgangsstoff ausführen. Man verwendet vorzugsweise einen Niederalkyl - wie Methyl - oder Aethyl - enoläther. Sodann kann man dieselbe Dehydrierung eines Enoläthers auch mit Mangandioxyd erlangen, vorzugsweise in einem halogenierten Kohlenwasserstoff, wie Chloroform oder Dichlormethan, wobei das Aetherbildende Alkyl abgespalten wird. Den als Ausgangsstoff zu verwendenden 3-Enoläther kann man nach allgemein bekannten Methoden erhalten, vorzugsweise durch Behandeln des gesättigten 3-Ketons mit einem entsprechenden Ameisensäureorthoester, wie Methylorthoformiat oder Aethylorthoformiat, unter Säurekatalyse,

wobei man Bedingungen wählt, bei denen die 9α,11α-Epoxygruppe nicht gespalten wird.

Ein anderes bevorzugtes Dehydrierungsmittel für die Einführung der 1,2-Doppelbindung ist das Selendioxyd und Derivate der selenigen Säure oder von aromatischen Seleninsäuren oder Selenenylhalogeniden. Die Dehydrierung mit Selendioxid wird z.B. in einem geeigneten Lösungsmittel, vorzugsweise einem tertiären niederaliphatischen Alkohol mit 1-7 C-Atomen, wie insbesondere tert.-Butylalkohol oder tert.-Amylalkohol, bei Temperaturen zwischen ca. -5° und ca. 150° ausgeführt, gegebenenfalls unter Zusatz einer tertiären Base, wie Pyridin oder Collidin. Ferner kann man nach der sogenannten "Selenoxid-Eliminationsmethode" nach Reich (Journal of the American Chemical Society 97, (1975), 5434) verfahren, indem man durch Behandlung des Ausgangsstoffes in Form eines Metallenolats oder eines Enolesters mit einem selenierenden Mittel, wie insbesondere einem Arylselenylhalogenid, z.B. Phenylselenylbromid - oder -chlorid, oder Benzolselenenyl-trifluoracetat, das 2-Arylseleno-3-keton herstellt, dieses mit Wasserstoffperoxid, Natriumperjodat, einem organischen Peroxid, wie m-Chlorperbenzoesäure, oder Ozon zum 2-Arylselenoxy-3-keton oxydiert, das schon bei tiefer Temperatur zum $\Delta^1$-3-Keton und Phenylseleneniger Säure fragmentiert.
Für die Ausführung dieser Methode werden vorzugsweise die in der oben angeführten Originalpublikation verwendeten Lösungsmittel verwendet und die dort angegebenen Bedingungen eingehalten, wobei besonders im Oxydationsschritt wegen der stark exothermischen Reaktion Vorsicht geboten ist.

Eine andere Methode bedient sich aromatischer Seleninsäuren oder ihrer Derivate, wie der Anhydride, z.B. Benzolseleninsäureanhydrid. Die Methode ist in J.C.S. Chem. Commun. 1978, 278-279 von T.G. Back beschrieben.

Sodann kann man die 1,2-Doppelbindung auch nach der in "Synthesis 11, 773-774, (1977)" von E. Mincione u.a. beschriebenen Methode mit

Palladiumsalzen, z.B. Palladiumchlorid, in einem geeigneten Lösungsmittel, z.B. in einem niederaliphatischen Alkohol mit 1-7 C-Atomen, wie insbesondere in Butylalkohol, einführen.

Ferner können die Methoden mit Sauerstoff in Gegenwart von metallischen Katalysatoren (J.Org. Chemistry, 36, 752 (1971) oder die Methode mit Pyridin-N-oxid und Acetanhydrid [J.Org. Chemistry, 38, 3737 (1973)] angewendet werden.

Gemäss der oben angeführten Verfahrensmethode c) geht man von einer Verbindung aus, die im Ring A eine Gruppierung enthält, welche man in die 3-Oxogruppe überführen kann; die Ueberführung erfolgt in an sich bekannter Weise. Die genannte Gruppierung ist z.B. ein funktionelles Derivat der 3-Ketonverbindungen gemäss Formel (VII) oder (VIII), worin $Z_1$ und $Z_2$ eine funktionell abgewandelte Oxogruppe bedeutet. Solche Ausgangsverbindungen sind z.B. Oxime, Hydrazone, Semicarbazone oder Ketimine oder Ketale, Thioketale oder Thiohemiketale. Die oben genannten stickstoffhaltigen funktionellen Derivate der Ketone können unsubstituiert sein, wie das freie Oxim, Hydrazon, Semicarbazon oder Ketimin oder können im funktionellen Teil z.B. durch Alkyl- oder Alkylenreste mit 1- bzw. 2-7 C-Atomen substituiert sein.

Ketal, Thioketal, Hemiketal- und Hemithioketalgruppen sind insbesondere solche, die sich von aliphatischen, vorzugsweise gesättigten, Alkoholen mit 1-7 C-Atomen ableiten, wie Methyl-, Aethyl-, Propylalkohol, den Butyl- oder Amylalkoholen, oder von analogen monocyclischen araliphatischen Alkoholen, wie Benzylalkohol. Besonders bevorzugte Ketale und Thioketale leiten sich von aliphatischen zweiwertigen Alkoholen mit 2-4 C-Atomen ab, wie Aethylenglykol oder Propylenglykol.

In den Ausgangsstoffen der Formeln (VII) und (VIII) kann $Z_1$ und $Z_2$ auch eine Enol- oder Enamingruppe sein, wobei in diesem Falle $Z_2$ eine 3,4-Doppelbindung darstellt und $Z_1$ für eine funktionelle abgewandelte Hydroxylgruppe steht. Vor allem handelt es sich bei diesen Enolderivaten um Enolester, Enoläther, Enamine und Metallenolate. Enolester leiten sich vorzugsweise von einer aliphatischen Carbonsäure mit 1-7 C-Atomen ab, wie von der Ameisen-, Essig-, Propionsäure, den Butter- oder Valeriansäuren oder von einer einfachen monocyclischen aromatischen Carbonsäure, wie der Benzoesäure oder einer ihrer Homologen oder Substitutionsprodukte. Enoläther leiten sich vorzugsweise von einem niederen aliphatischen Alkohol, z.B. einem der oben genannten, ab. Enamine leiten sich vorzugsweise von einem primären oder sekundären aliphatischen Amin mit 1-7 C-Atomen oder einem einfachen monocyclischen aromatischen oder araliphatischen Amin ab. Als Beispiele dieser Amine seien etwa folgende aufgeführt: Methyl-, Propyl-, Isopropyl-, n-Butyl-, iso-Butyl- oder tert.-Butylamin, Pyrrolidin, Piperidin, Anilin, oder die Toluidine und Benzylamin. Metallenolatgruppen sind z.B. diejenigen von Natrium-, Kalium- oder Lithiumenolate.

Die Gruppe $Z_1$ und $Z_2$ kann auch eine andere beliebige Ketonschutzgruppe sein, die z.B. durch Solvolyse in die freie 3-Oxogruppe umgewandelt werden kann.

Eine Gruppe $Z_1$ und $Z_2$ kann schliesslich auch eine freie oder funktionell abgewandelte Hydroxy-, Thiol- oder Aminogruppe zusammen mit Wasserstoff darstellen, die während der durchzuführenden Oxydationsreaktion zur 3-Oxogruppe leicht verseift und intermediär die freie 3-Hydroxygruppe bildet.

Die verfahrensgemässe Umwandlung einer Gruppe $Z_1$ und $Z_2$ in eine Oxogruppe kann in an sich bekannter Weise geschehen. So können funktionelle Derivate der 3-Ketone, wie die oben genannten Oxime, Hydrazone, Semicarbazone, Ketimine, Ketale, Thioketale, Hemiketale und Hemithioketale unter solvolytisch, insbesondere sauren Bedingungen, z.B. mit verdünnter Mineralsäure, wie Chlorwasserstoff- oder Schwefelsäure, Perchlorsäure, Oxalsäure oder einer Sulfonsäure, wie insbesondere einer niederen aliphatischen,

wie Methansulfonsäure oder einer monocyclischen aromatischen, wie p-Toluolsulfonsäure, oder mit relativ starken organischen Säuren, wie Oxal- oder Trifluoressigsäure, in die Ketone übergeführt werden. Ebenfalls solvolytisch, insbesondere wiederum unter dem Einfluss von Säuren, aber gegebenenfalls auch basisch, können auch Enolderivate in die Ketone umgewandelt werden. Es müssen dabei Bedingungen gewählt werden, bei denen die Epoxygruppe unter dem Einfluss der Säure nicht gespalten wird.

Bei der Ueberführung von Thioketalen in die 3-Ketone arbeitet man vorzugsweise unter Zusatz einer Schwefel-bindenden Verbindung, z.B. eines Metallsalzes, insbesondere eines Schwermetallsalzes, wie Cadmiumcarbonat und/oder Quecksilber-(II)-chlorid. Da das letztgenannte Mittel selber in Anwesenheit von Wasser stark sauer reagiert, ist bei seiner Anwendung keine zusätzliche Säure als Katalysator notwendig. Besonders gut gelingt auch die Entthioketalisierung durch Behandlung mit Methyljodid in Aceton bei Siedetemperatur oder im Bombenrohr bei ca. 60°.

Die Oxidation einer Hydroxy, Thiol- oder Aminogruppe $Z_1$ oder $Z_2$ geschieht ebenfalls in an sich bekannter Weise, z.B. mittels Verbindungen des 6-wertigen Chroms, in saurer oder leicht alkalischer Lösung. So kann man z.B. mit Chromtrioxid in Gegenwart von Schwefelsäure und gegebenenfalls Aceton oder Eisessig oder mit Chromtrioxyd in Gegenwart einer tertiären aromatischen Base, insbesondere Pyridin, oxydieren, sofern Bedingungen gewählt werden, die die 9α,11α-Epoxygruppe intakt lassen.

Die Oxidation der 3-Hydroxygruppe in den 1-Dehydro-steroiden kann aber auch mit einem Aluminium-Alkoholat und einem Keton, z.B. nach Oppenauer, vorgenommen werden, z.B. in Toluol, oder mit Mangandioxyd, Bleitetraacetat, mit dehydrierenden Chinonen, wie 2,3-Dichlordicyanobenzochinon, mit unterchloriger Säure oder ihren Derivaten, z.B. mit Bromacetamid oder Bromsuccinimid, mit Wismuthtrioxid,

Sauerstoff in alkalischer Lösung, Kupferacetat und ähnlichen Kupfersalzen. Besonders gut verläut die Oxydation mit Mangandioxyd in Isopropylalkohol bei Raumtemperatur.

Ist $Z_1$ oder $Z_2$ bei der Methode c) eine veresterte oder verätherte Hydroxy- oder Thiolgruppe, oder eine acylierte Aminogruppe, so werden vorzugsweise die freien funktionellen Gruppen gebildet und diese wie oben beschrieben zur Oxogruppe oxydiert. In mehreren Fällen gelingt auch die direkte Oxydation solcher funktionell modifizierter Hydroxygruppen, namentlich wenn in saurer Lösung gearbeitet wird, da dann intermediär die freien funktionellen Gruppen entstehen.

Nach der Methode d) des Verfahrens der vorliegenden Erfindung wird der Spirolacton- oder Spiroätherring in 17-Stellung aus $\Delta^1$-17α-Hydroxy-androsten-Verbindungen der Formel (IX), welche in 17α-Stellung einen substituierten Kohlenwasserstoff aufweisen, der zur Ringbildung mit der 17β-Hydroxygruppe befähig ist, hergestellt.

Der Ringschluss zum Spiroäther in einer Verbindung der Formel(IX) in welcher W die Gruppe

$$\cdots CH_2-CH_2-CH_2-OH$$

darstellt, kann mittels eines Sulfonsäurechlorids in einer tertiären aromatischen Base, wie insbesondere p-Toluolsulfonsäurechlorid in Pyridin, oder analoger Reaktionspartner, erfolgen. [Siehe z.B. J. Med. Chem. 6, 617-618 (1963); U.S. Patent Nr. 3,798,213; Tetrahedron Letters (1970), 5057-5059]. Die entsprechenden Ausgangsstoffe können nach Methoden, die in den eben zitierten Artikeln beschrieben wurden, hergestellt werden, wobei selbstverständlich die $\Delta^1$-3-Oxogruppe intermediär geschützt werden muss. Eine günstigere Methode zur

Bildung des genannten Spiroäthers ist in der Deutschen Offenlegungsschrift 2 617 295 beschrieben:
Sie besteht darin, dass man Verbindungen der Formel (IX), die in 17α-Stellung einen Substituenten W der Formel

$$...CH_2-CH_2-CH_2-R_o$$

worin $R_o$ eine Diniederalkylaminogruppe bedeutet, aufweisen, unter Ringschluss desaminiert. Die Desaminierung erfolgt z.B. durch thermische Zersetzung der quaternären Basen der genannten Ausgangsstoffe. In der "Diniederalkylaminogruppe" haben die Alkylreste höchstens 7 C-Atome und sind z.B. Methyl, Aethyl, n-Propyl-, i-Propyl-, n-Butyl-, sec.-Butyl-, tert.-Butyl, ein verzweigter oder vorzugsweise gerader Pentyl-, Hexyl- oder Heptylrest. Die Desaminierungs-Reaktion führt man unter den an sich bekannten Bedingungen der Hofmannschen Eliminierung durch, indem man die Diniederalkylaminoverbindung in das entsprechende quaternäre Triniederalkylammoniumsalz umwandelt und dieses in Form der entsprechenden quaternären Base thermisch zersetzt. Als Quaternisierungsmittel ist ein Diniederalkylsulfat oder insbesondere ein Niederalkylhalogenid, wie Niederalkylchlorid, Niederalkylbromid und vorzugsweise Niederalkyljodid, geeignet. Besonders ist eine Niederalkylgruppe die Methylgruppe. Die Quaternisierung wird in überschüssigem Alkylierungsmittel, oder vorteilhaft in einem organischen Lösungsmittel, insbesondere einem Niederalkanol, vor allem Methanol, aber auch Aceton, Methyläthylketon oder Aethylacetat ausgeführt. Die quaternäre Base wird aus dem entsprechenden Salz mittels einer starken Base freigesetzt. Zu diesem Zweck verwendet man stark basische Ionenaustauscher, Silberhydroxid, Thallium(I)-hydroxid, und vor allem Alkalimetallhydroxide, wie Natrium- oder Kaliumhydroxid. Im Falle von Sulfaten kann man auch Bariumhydroxid verwenden. Als Lösungs- oder Verdünnungsmittel dient Wasser, gegebenenfalls in Anwesenheit eines

organischen, mit Wasser mischbaren Lösungsmittel, z.B. eines Alkohols, wie eines Niederalkanols, eines niederen Glykols oder des Glycerins. Man kann aber die Base auch mit einem Kalium- oder insbesondere Natriumalkoholat, z.B. einem von den bereits erwähnten Alkoholen abgeleiteten, freisetzen. Die Thermolyse führt man praktisch z.B. so aus, dass man eine erhaltene Lösung der quaternären Base bei steigender Temperatur, und gegebenenfalls unter vermindertem Druck, konzentriert und, wenn notwendig, bis zum Zersetzungspunkt, beispielsweise auf ca. 200°, erwärmt. Eine besonders vorteilhafte Variante besteht darin, dass man das quaternäre Salz mit einer äquimolaren Menge Alkalimetallhydroxid in wässriger Lösung versetzt, Aethylenglykol zufügt und das Gemisch bis zur Vollendung der Zersetzung durch langsame Destillation konzentriert. Es ist empfehlenswert, freie quaternäre Basen nur unter sorgfältigem Ausschluss des atmosphärischen Kohlendioxyds zu verarbeiten. Weitere Angaben bezüglich dieser Verfahrensmethode sowie insbesondere auch über die Herstellung der Ausgangsstoffe sind in der oben erwähnten Offenlegungsschrift wiedergegeben. Bei der Herstellung der Ausgangsstoffe wird die $\Delta^1$-3-Oxogruppe intermediär geschützt.

Verbindungen der Formel (I), in welchen X eine Oxogruppe bedeutet, können nach der obigen Variante d) aus Verbindungen der Formel (IX), worin W eine freie, veresterte oder amidierte Carboxyäthylgruppe bedeutet, hergestellt werden.

Ausgangsverbindungen für dieses Verfahren mit einer freien Carboxylgruppe können gemäss der oben schon erwähnten und noch zu besprechenden Variante e) für die Verfahrensprodukte gemäss der Erfindung erhalten werden. Sie cyclisieren schon in Gegenwart katalytischer Mengen von Säuren zu den genannten Spirolactonen. Auch die Ester gehen diese Reaktion ein. Als Ausgangsstoffe mit amidierter Carboxylgruppe werden vor allem Diniederalkylamide (wobei die Alkylreste vorzugsweise 1-7 C-Atome aufweisen, jedoch in erster Linie Methyl darstellen), verwendet. Solche Verbindungen können zu den Spirolactonen z.B. gemäss dem in der Deutschen Offenlegungsschrift 24 24 752 beschriebenen

Verfahren durch Behandlung mit einem sauren Kationenaustauscher in die Verfahrensprodukte übergeführt werden. Man löst den Ausgangsstoff in einem geeigneten Lösungsmittel, wie etwa einem aromatischen Kohlenwasserstoff, z.B. Benzol, Toluol, Xylol, einem Aether, wie Diäthyläther, Dibutyläther, Dioxan, Tetrahydrofuran oder Diäthylenglykoldimethyläther, einem halogenierten Kohlenwasserstoff, wie Methylenchlorid, Chloroform oder Dichloräthylen, aber auch in Aethylacetat, Pyridin, Dimethylformamid, Dimethylsulfoxid, in Alkoholen, wie Methanol, Aethanol, oder Ketonen, wie Aceton oder Methyl-äthyl-keton, und bringt es mit dem sauren Kationenaustauscher zusammen. Die Reaktion ist im allgemeinen bei Raumtemperatur nach 5-25 Stunden beendet. Sie kann aber auch bei erhöhter Temperatur durchgeführt werden, so dass sie sich gegebenenfalls auf nur ca. 2 Stunden verkürzt.
Die Ausgangsstoffe lassen sich nach in der oben genannten Patentschrift erwähnten Methoden herstellen.

Gemäss Verfahrensvariante e) werden die Verbindungen der Formel (X) zu Verbindungen der Formel (I) oder (II) oxydiert. Die Oxydation kann in einer Stufe bis zur Carbonsäure bzw. dem Spirolacton mittels starker Oxydationsmittel, wie Chromsäure oder ihrer Derivate, z.B. Chromtrioxid in saurer Lösung, z.B. in Schwefelsäure, gegebenenfalls in Anwesenheit von Aceton und/oder Essigsäure, oder in basischer Lösung, z.B. Chromtrioxid-Pyridin, erfolgen; oder sie kann stufenweise geschehen, indem zuerst der entsprechende Aldehyd nach an sich bekannten Methoden, z.B. über die entsprechenden Nitrone, oder mit Kupfer(II)-salzen in alkalischer Lösung, hergestellt wird, und dieser weiter in an sich bekannter Weise zur Säure oxydiert wird, was in an sich bekannter Weise z.B. mit Chrom(VI)-Verbindungen, durchgeführt werden kann.

Gemäss Verfahrensvariante f) werden z.B. Verbindungen der Formel (XI), in denen V = H ist, zum entsprechenden Spirolacton oxydiert. Dies kann vorteilhafterweise in an sich bekannter Weise mit Rutheniumtetroxid [Journal of the American Chemical Society 80, 6682 (1958), J. Organic Chemistry 28, 2729 (1963)] oder t-Butylchromat (Tetrahedron Letters No. 58, 5057-5059) erzielt werden.

Gemäss derselben Variante können aber auch Verbindungen der Formel (XI) verwendet werden, in welchen V eine freie, veresterte oder verätherte Hydroxygruppe bedeutet. Solche Verbindungen stellen Hemiacetale oder Hemithioacetale der Aldehyde der Formel

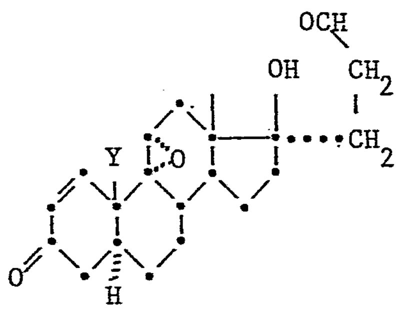

und ihre Ester und Aether, dar. Diese werden, gemäss dem Verfahren der Variante f), in an sich bekannter Weise zu den Verbindungen der Formel I oxydiert, worin X = 0 ist.
Die Oxydation kann mit solchen Oxydationsmitteln ausgeführt werden, welche imstande sind, in saurer Lösung einen Aldehyd zur entsprechenden Carbonsäure zu oxidieren, wobei Bedingungen einzuhalten sind, unter denen die 9α,11α-Expoxygruppe nicht gespalten wird. Besonders geeignet sind Chromtrioxid in saurer Lösung, insbesondere in niederen Alkancarbonsäuren als Lösungsmittel, oder Chromschwefelsäure in Aceton, ferner Salpetersäure oder salpetrige Säure, bzw. Stickoxide, Hypohalogenite, insbesondere unterchlorige oder unterbromige Säure, N-Bromsuccinimid oder N-Chlorsuccinimid in saurer Lösung, Permanganat oder Persäuren, wie Peressigsäure, Perbenzoesäure, m-Chlorperbenzoesäure oder Perphthalsäure. Die Oxydation wird im allgemeinen in inerten organischen Lösungsmitteln durchgeführt, wie in niederen aliphatischen Carbonsäuren, Ketonen, Aethern, Dimethylsulfoxid, oder chlorierten Kohlenwasserstoffen, z.B. Methylenchlorid, Chloroform oder Tetrachlorkohlenstoff. Man kann auch Wasser zusetzen, und dies ist von Vorteil, wenn Salpetersäure, salpetrige Säure oder Kaliumpermangenat als Oxydationsmittel verwendet werden. Die Temperaturen werden zwischen ca. 0 und 80° gehalten. Die Ausgangsstoffe können analog den bekannten Verfahren zur Herstellung entsprechender in 9,11-Stellung unsubstituierten Verbindungen z.B. wie in folgenden Patentschriften beschrieben:

| | | |
|---|---|---|
| Deutsche | Offenlegungsschrift | 2 237 143 |
| " | " | 2 248 834 |
| " | " | 2 248 835 |
| " | " | 2 251 476 |
| " | " | 2 625 723. |

hergestellt werden.

Wie z.B. in der Offenlegungsschrift 2 237 143 angegeben, können die Cyclohalbacetale - oder thioacetale, die als Ausgangsstoffe zu verwenden sind und die bereits in den Ringen A und B gemäss Formel XI substituiert sind, durch Umsetzung eines entsprechenden 17-Ketons, welches im A-Ring eine in die $\Delta^1$-3-Oxo-Gruppe überführbare Gruppe enthält, mit einem Organometallderivat der Formel

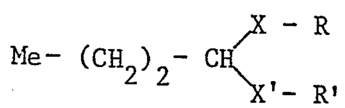

(Me = z.B. Alkalimetall)

worin X und X' Sauerstoff- oder Schwefel und R und R' einen Kohlenwasserstoffrest mit 1-20 C-Atomen, vorzugsweise 1-10 C-Atomen, bedeuten, worin R und R' auch miteinander verbunden sein können, so dass sie zusammen mit den Atomen X und X' und der -CH-Gruppe einen Ring bilden, in welchem Falle R und R' vorzugsweise 2-6 C-Atome enthalten, und Solvolyse des erhaltenen Kondensationsproduktes, hergestellt werden. In erhaltenen freien Cyclohalbacetalen kann die Hydroxygruppe schon gleichzeitig mit dem Solvolyse-Schritt oder anschliessend an diesen verestert oder veräthert werden, was durch Zugabe eines Alkohols, z.B. eines der oben genannten niederen aliphatischen Alkohole, geschieht. Die Aether-Bildung wird von geringen Säure-Mengen katalysiert. Auch die Veresterung der erhaltenen Aldehydhalbacetale tritt leicht ein, wenn man sie mit der gewünschten Säure in Kontakt bringt. So können z.B. durch Behandlung mit Essigsäure oder Thioessigsäure bei Temperaturen zwischen ca. Raumtemperatur und 100° die Acetate bzw. Thioacetate der cyclischen Halbacetale hergestellt werden.

Nach der Kondensation des 17-Ketons mit der genannten Organometallverbindung und gegebenenfalls nach der besprochenen Solvolyse und/oder Veresterung oder Verätherung kann aus der genannten in die $\Delta^1$-3-Oxogruppe überführbare Gruppe dieselbe gebildet werden.

Gemäss Variante g) wird aus Verbindungen der Formeln (XII) oder (XIII), in welchen in 1- oder 2-Stellung eine Abgangsgruppe $T_1$ oder $T_2$ vorhanden ist, dieselbe unter Ausbildung einer Doppelbindung abgespalten. Eine solche Abgangsgruppe ist insbesondere eine freie oder veresterte Hydroxy- oder Mercaptogruppe, oder ein Halogen, insbesondere Brom, Chlor oder Jod. 1α- oder 2α-Hydroxyverbindungen können in an sich bekannter WEise, z.B. auf mikrobiologischem Wege hergestellt werden. Besonders günstige Abgangsgruppen sind mit einer Carbonsäure- oder insbesondere mit einer Sulfonsäure, z.B. einer aliphatischen Sulfonsäure mit 1-7 C-Atomen oder einer monocyclischen aromatischen Sulfonsäure, wie Methansulfonsäure, Benzol- oder p-Toluolsulfonsäure, veresterte Hydroxygruppen. Die Abspaltung einer solchen Gruppe erfolgt z.B. durch Einwirkung eines basischen Reagenz, wie insbesondere einer tertiären Base, wie Pyridin oder Collidin, oder einer anorganischen Base, insbesondere eines basisch wirkenden Salzes einer niederaliphatischen Carbonsäure, wie Natriumacetat. Aus einer freien 1-Hydroxy- oder 2-Hydroxyverbindung kann die Doppelbindung in 1,2 Stellung unter den Bedingungen der Oppenauer Oxidation, z.B. bei der Oxidation eines 1,3-Diols, gebildet werden.

Eine besonders günstige Variante dieser Methode geht von Ausgangsstoffen der Formeln XII oder XIII aus, in welchen $T_1$ ein

Halogenatom und $T_2$ = H ist. $T_1$ ist in diesem Falle insbesondere Chlor oder Jod, in erster Linie aber Brom. Solche Verbindungen erhält man durch Halogenierung der entsprechenden unsubstituierten Verbindungen z.B. mit elementarem Halogen, z.B. Brom, oder mit geeigneten Brom abgebenden Mitteln, wie z.B. Perbromide von organischen tertiären Stickstoffbasen oder von Aethern, wie Pyridinperbromid oder Dioxanperbromid.

Die Dehydrohalogenierung der solchermassen halogenierten Ausgangsstoffe erfolgt mit basischen Mitteln, wie z.B. tertiären aromatischen Basen, wie Pyridin oder Chinolin, oder ihren Homologen, wie Collidin, oder aliphatischen Basen, oder cycloaliphatischen Basen, wie Triniederalkyl-aminen, z.B. der sogenannten Hünigschen Base, dem Aethyldiisopropylamin. Man kann die Reaktion in einem inerten Lösungsmittel ausführen, z.B. in einem chlorierten aliphatischen Kohlenwasserstoff, z.B. Methylenchlorid, Aethylenchlorid, Tetrachlorkohlenstoff oder Chloroform, oder in einem Keton, wie Aceton oder Methylpropylketon oder Cyclohexanon, oder in einem Ester, wie Aethylacetat oder einem Aether, wie Dioxan oder Tetrahydrofuran, bei Temperaturen zwischen Raumtemperatur und ca. 150°. Man kann die Dehydrohalogenierung aber auch mit anorganischen Basen, wie insbesondere mit basischen Salzen von Alkali- oder Erdalkalimetallen oder von Magnesium, z.B. Kalium-, Natrium- oder Ammoniumsalzen von aliphatischen Carbonsäuren mit 1-7 C-Atomen, wie solchen der Essigsäure, Propionsäure, den Buttersäuren oder Valeriansäuren, oder Lithium, und insbesondere auch mit einem Lithiumhalogenid, wie Lithiumchlorid oder -bromid in Gegenwart eines Alkali- oder Erdalkalicarbonats, wie Calciumcarbonat, bewirken. Als Lösungsmittel kann auch hier eine der oben hervorgehobenen verwendet werden, wie ein Aether, z.B. Tetrahydrofuran, aber auch ein niederes aliphatisches Dialkylamid, wie Dimethylacetamid oder ein Sulfoxid, wie Dimethylsulfoxid. Bei Verwendung von basischen Salzen der Alkalien oder Erdalkalien, die sich von organischen Säuren ableiten, verwendet man als Reaktions- bzw. Lösungsmittel vorteilhaft die entsprechende organische Säure,

z.B. Essigsäure, wenn Natriumacetat verwendet wird. (Vgl. z.B. US-Patent 3 076 001 - Gazz. Chim. Ital. 93, 10705 (1963).

Die funktionelle Abwandlung einer Hydroxylgruppe oder einer Carboxylgruppe gemäss Verfahrensvariante h) kann ebenfalls in an sich bekannter Weise ausgeführt werden. Geeignete Mittel zur Verätherung der Hydroxygruppe sind beispielsweise Diazoverbindungen, wie gegebenenfalls substituierte Diazoniederalkane, z.B. Diazomethan, Diazoäthan, Diazo-n-butan. Diese Reagenzien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z.B. Methylenchlorids, oder eines Aethers, wie eines Diniederalkyläthers, z.B. Diäthyläthers, oder eines cyclischen Aethers, z.B. Tetrahydrofurans, angewendet.

Von den bekannten, im folgenden erwähnten Verätherungsmethoden sind jene geeignet, welche unter Bedingungen verwendet werden können, die die 9α,11α-Epoxidgruppe intakt lassen. Als Verätherungsmittel kommen insbesondere Ester entsprechender Alkohole mit starken anorganischen oder organischen Säuren, wie Mineralsäuren, z.B. Halogenwasserstoffsäuren, wie Chlorwasserstoff-, Bromwasserstoff- oder Jodwasserstoffsäure, ferner Schwefelsäure, oder Halogenschwefelsäure, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls, durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure, in Betracht. Solche Ester sind u.a. Niederalkylhalogenide, Diniederalkylsulfate, wie Dimethylsulfat, ferner Fluorsulfonsäureester, wie -niederalkylester, z.B. Flurosulfonsäuremethylester, oder gegebenenfalls Halogen-substituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z.B. Methylenchlorid, eines Aethers, wie Dioxan oder Tetrahydrofuran, oder eines Gemisches verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organische Basen, wie, vorzugsweise sterisch gehinderte, Triniederalkylamine, z.B. N,N-Diisopropyl-N-äthyl-amin (vorzugsweise zusammen mit Halogensulfonsäure-niederalkylestern oder gegebenenfalls halogensubstituierten Methansulfonsäure-niederalkylestern) an, wobei unter Kühlen, bei Raumtemperatur oder unter Erwärmen, z.B. bei Temperaturen von etwa -20°C bis etwa 50°C und, wenn notwendig, in einem geschlossenen Gefäss und/oder in einer Inertgasatmosphäre, z.B. Stickstoffatmosphäre, gearbeitet wird.

Durch Phasentransfer-Katalyse [siehe Dehmlow, Angewandte Chemie, Bd. 5, S. 187 (1974)] kann die oben beschriebene Verätherungsreaktion wesentlich beschleunigt werden. Als Phasentransfer-Katalysatoren können quaternäre Phosphoniumsalze und insbesondere quaternäre Ammoniumsalze, wie gegebenenfalls substituierte Tetraalkylammoniumhalogenide, z.B. Tetrabutylammoniumchlorid, -bromid oder -jodid, oder auch Benzyl-triäthylammoniumchlorid, in katalytischen oder bis zu äquimolaren Mengen verwendet werden. Als organische Phase kann irgendein der mit Wasser nicht mischbaren Lösungsmittel dienen, beispielsweise einer der gegebenenfalls halogenierten, wie chlorierten niederen aliphatischen, cycloaliphatischen oder aromatischen Kohlenwasserstoffe, wie Tri- oder Tetrachloräthylen, Tetrachloräthan, Tetrachlorkohlenstoff, Chlorbenzol, Toluol oder Xylol. Die als Kondensationsmittel geeigneten Alkalimetallcarbonate oder -hydrogencarbonate, z.B. Kalium- oder Natriumcarbonat oder -hydrogencarbonat, Alkalimetallphosphate, z.B. Kaliumphosphat, und Alkalimetallhydroxide, z.B. Natriumhydroxid, können der Reaktionsmischung titrimetrisch, z.B. mittels eines Titrierautomaten,

zugesetzt werden, damit der pH-Wert während der Verätherung zwischen etwa 7 und etwa 8.5 bleibt.

Weitere Verätherungsmittel sind geeignete Acetal-Verbindungen, z.B. gem-Di-niederalkoxy-niederalkane, wie 2,2-Dimethoxy-propan, die in Gegenwart von starken organischen Sulfonsäuren, wie p-Toluolsulfonsäure, und eines geeigneten Lösungsmittel, wie eines Diniederalkyl- oder Niederalkylensulfoxids, z.B. Dimethylsulfoxid, zur Anwendung gelangen, oder geeignete Orthoester, z.B. Orthoameisensäuretriniederalkylester, z.B. Orthoameisensäure-triäthylester, die in Gegenwart einer starken Mineralsäure, z.B. Schwefelsäure, oder einer starken organischen Sulfonsäure, wie p-Toluolsulfonsäure, und eines geeigneten Lösungsmittels, wie eines Aethers, z.B. Dioxan, verwendet werden.

Weitere Verätherungsmittel sind entsprechende trisubstituierte Oxoniumsalze (sogenannte Meerweinsalze), oder disubstituierte Carbenium- oder Haloniumsalze, worin die Substituenten die veräthernden Reste sind, beispielsweise Triniederalkyloxoniumsalze, sowie Diniederalkoxycarbenium- oder Diniederalkylhaloniumsalze, insbesondere die entsprechenden Salze mit komplexen fluorhaltigen Säuren, wie die entsprechenden Tetrafluorborate, Hexafluorphosphate, Hexafluorantimonate oder Hexachlorantimonate. Solche Reagentien sind z.B. Trimethyloxonium- oder Triäthyloxonium-hexafluorantimonat, -hexachlorantimonat, -hexafluorphosphat oder -tetrafluorborat, Dimethoxycarbeniumhexafluorphosphat oder Dimethylbromoniumhexafluorantimonat. Man verwendet diese Verätherungsmittel vorzugsweise in einem inerten Lösungsmittel, wie einem Aether oder einem halogenierten Kohlenwasserstoff, z.B. Diäthyläther, Tetrahydrofuran oder Methylenchlorid, oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-äthyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgasatmosphäre, z.B. Stickstoffatmosphäre.

Weitere Verätherungsmittel sind schliesslich entsprechende 1-substituierte 3-Aryltriazenverbindungen, worin der Substituent den veräthernden Rest, und Aryl vorzugsweise gegebenenfalls substituiertes Phenyl, z.B. Niederalkylphenyl, wie 4-Methylphenyl, bedeutet. Solche Triazenverbindungen sind 3-Aryl-1-niederalkyltriazene, z.B. 3-(4-Methylphenyl)-1-methyl-triazen, 3-(4-Methylphenyl)-1-äthyl-triazen oder 3-(4-methylphenyl)-1-isopropyl-triazen. Diese Reagentien werden üblicherweise in Gegenwart von inerten Lösungsmitteln, wie gegebenenfalls halogenierten Kohlenwasserstoffen oder Aethern, z.B. Benzol, oder Lösungsmittelgemischen, und unter Kühlen, bei Raumtemperatur und vorzugsweise bei erhöhter Temperatur, z.B. bei etwa 20°C bis etwa 100°C, wenn notwendig, in einem geschlossenen Gefäss und/oder in einer Inertgas-, z.B. Stickstoffatmosphäre verwendet.

Zur Veresterung der Hydroxygruppe behandelt man das Ausgangsmaterial der Formel (IV) mit einem den gewünschten Acylrest einer organischen Carbonsäure einführenden Acylierungsmittel unter Bedingungen, die die 9α,11α-Epoxydgruppe intakt lassen. Dabei verwendet man die entsprechende Carbonsäure oder ein reaktionsfähiges Derivat davon, insbesondere ein Anhydrid, inkl. ein gemischtes oder inneres Anhydrid einer solchen Säure. Gemischte Anhydride sind z.B. diejenigen mit Halogenwasserstoffsäuren, d.h. die entsprechenden Säurehalogenide, insbesondere -chloride, ferner mit Cyanwasserstoffsäure, oder dann diejenigen mit geeigneten Kohlensäurehalbderivaten, wie entsprechenden -halbestern (wie die z.B. mit einem Halogen-ameisensäure-niederalkyl, wie Chlorameisensäure-äthylester oder -isobutylester, gebildeten gemischten Anhydride) oder mit gegebenenfalls substituierten, z.B. Halogen, wie Chlor, enthaltenden Niederalkancarbonsäuren (wie die mit Pivalinsäurechlorid oder Trichloressigsäurechlorid gebildeten gemischten Anhydride). Innere Anhydride sind z.B. diejenigen von organischen

Carbonsäuren, d.h. Ketene, wie Keten oder Diketen, oder diejenigen von Carbamin- oder Thiocarbaminsäuren, d.h. Isocyanate oder Isothiocyanate. Weitere reaktionsfähige, als Acylierungsmittel verwendbare Derivate von organischen Carbonsäuren sind aktivierte Ester, wie geeignet substituierte Niederalkylester, z.B. Cyanmethylester, oder geeignet substituierte Phenylester, z.B. Pentachlorphenyl- oder 4-Nitrophenylester. Die Veresterung kann, wenn notwendig, in Gegenwart von geeigneten Kondensationsmitteln, bei Verwendung von freien Carbonsäuren, z.B. in Gegenwart von Carbodiimidverbindungen, wie Dicyclohexylcarbodiimid, oder Carbonylverbindungen, wie Diimidazolylcarbonyl, und bei Verwendung von reaktionsfähigen Säurederivaten z.B. in Gegenwart von basischen Mitteln, wie Triniederalkylaminen, z.B. Triäthylamin, oder heterocyclischen Basen, z.B. Pyridin oder 4-Dimethylaminopyridin, durchgeführt werden. Die Acylierungsreaktion kann in Abwesenheit oder in Gegenwart eines Lösungsmittels oder Lösungsmittelgemisches, unter Kühlen, bei Raumtemperatur oder unter Erwärmen, und, wenn notwendig, in einem geschlossenen Gefäss und/oder in einer Inertgas-, z.B. Stickstoffatomosphäre, durchgeführt werden. Geeignete Lösungsmittel sind z.B. gegebenenfalls substituierte, insbesondere gegebenenfalls chlorierte, aliphatische, cycloaliphatische oder aromatische Kohlenwasserstoffe, wie Benzol oder Toluol, wobei man geeignete Veresterungsreagentien, wie Essigsäureanhydrid, auch als Verdünnungsmittel verwenden kann.

Die Verbindungen der Formel (I) worin X = O ist, können in an sich bekannter Weise in Carbonsäuren gemäss der Formel (II) bzw. nach Verfahrensvariante i) in ihre Salze umgewandelt werden, indem man sie mit einer Base, z.B. einer Alkali- oder Erdalkalibase behandelt. Aus den zunächst durch die basische Behandlung erhaltenen Carbonsäuresalzen kann man, wenn erwünscht, die freien Säuren durch Ansäurern freisetzen. Als Alkali- bzw. Erdalkalibasen verwendet man z.B. entsprechende Hydroxide, wie Natrium- und insbesondere Kaliumhydroxid, Carbonate, wie Natrium- oder Kaliumcarbonat und oder Natrium- oder Kaliumhydrogencarbonat; als Reaktionsmedium verwendet man zweckmässig Gemische von

Wasser mit einem oder mehreren organischen Lösungsmitteln, vorzugsweise mit solchen, die mit Wasser mischbar sind, z.B. mit Niederalkanolen, wie Methanol, Aethanol oder Isopropylalkohol, mit cyclischen Aethern, wie Tetrahydrofuran oder Dioxan, mit Niederalkanonen, wie Aceton oder 2-Butanon, oder mit Niederalkylamiden niederer aliphatischer Säuren, unter diesen insbesondere N,N-Dimethylformamid. Vorzugsweise verwendet man nicht mehr als eine Aequivalent-Menge Base und vermeidet energische Reaktionsbedingungen. Dieselben Reaktionsbedingungen werden auch bei der Ueberführung einer nach irgendeinem Verfahren erhaltenen Carbonsäure gemäss Formel (II) in ihre Metallsalze angewendet. Zur Herstellung von Salzen mit organischen Basen werden ähnliche an sich bekannte Methoden benutzt, z.B. die Carbonsäure wird in wässerigem oder in organisch-wässerigem Medium mit einem Aequivalent der betreffenden Base bei Raumtemperatur oder leicht erhöhter Temperatur versetzt, und das Salz nach bekannten Methoden, z.B. durch Ausfällen oder Gefriertrocknung isoliert.

Die nach irgendeinem der Verfahren a) - i) erhaltenen Salze, wie die Alkali- oder Erdalkalimetallsalze, können in die entsprechenden freien 17β-Hydroxy-21-pregnen-carbonsäuren umgewandelt werden, indem man eine Lösung bzw. Suspension des Salzes in Wasser oder einem Wasser enthaltenden organischen Lösungsmittel ansäuert. Freie 17β-Hydroxy-21-pregnen-carbonsäuren gemäss Formel II kann man auch gewünschtenfalls mit einer entsprechenden Base in Salze umwandeln: auf diese Weise werden besonders Ammoniumsalze und Salze organischer Basen, z.B. der eingangs genannten, hergestellt.

Die für die oben beschriebenen Verfahrensvarianten benötigten Ausgangsstoffe können in an sich bekannter Weise hergestellt werden. Verbindungen der Formeln (III) und (IV) können durch Dehydratisierung von entsprechenden in 9,11-gesättigten Verbindungen erhalten werden, welche in 11-Stellung eine α- oder β-Hydroxygruppe aufweisen. Die Dehydratisierung derartiger 11α- oder 11β-Hydroxy-(5α)-20-spirox-1-en-3-one oder -3,21-dione oder der 11β,17-Dihydroxy-3-oxo-5α,17α-pregn-1-en-

21-carbonsäure oder der entsprechenden isomeren 11α-Hydroxy-21-carbonsäure und deren funktionellen Derivaten und des 11β,17β-Dihydroxy-17α-(3-hydroxypropyl)-5α-androst-1-en-3-ons und dessen funktionellen Derivaten kann nach einem beliebigen für Steroide der Pregnan- oder Androstanreihe bekannten Verfahren bewirkt werden, z.B. mit einer organischen Stickstoffbase, wie Pyridin oder Kollidin, in Gegenwart eines aliphatischen oder aromatischen Sulfonsäurehalogenids, wie z.B. p-Toluolsulfonylchlorid, bei Raumtemperatur oder etwas erniedrigter Temperatur. Ein vorteilhaftes Reagens zur Abspaltung der genannten 11β- oder 11α-Hydroxylgruppe unter Bildung der 9,11-Doppelbindung ist das Piperidino-schwefeltrifluorid, das vorzugsweise bei tiefer Temperatur, z.B. zwischen -40° und -60°, in einem inerten Lösungsmittel, wie einem chlorierten aliphatischen Kohlenwasserstoff, zur Anwendung gelangt.

Die oben genannten 11α- oder 11β-Hydroxy-Derivate ihrerseits können aus entsprechenden Verbindungen, welche in 1,2-Stellung gesättigt sind, nach denselben Verfahren wie oben für die Verfahrensvariante b) beschrieben, erhalten werden, z.B. durch Bromierung und Dehydrobromierung. Die soeben genannten gesättigten Derivate ihrerseits sind nach einer der für Spirolactone und Spiroxene bekannten Herstellungsmethoden, ausgehend von analogen 5α-H-Ausgangsstoffen, die in 11-Stellung bereits eine Sauerstofffunktion, insbesondere eine 11α- oder 11β-Hydroxygruppe und in 3-Stellung eine Hydroxygruppe oder eine andere in die 3-Ketogruppe überführbare Gruppe aufweisen, zugänglich. So kann z.B. 3,11α- oder 3,11β-Di-hydroxy-5α-androstan-17-on in Gegenwart von Lithium oder Magnesium mit einem γ-Chlorpropionaldehydacetal der Formel

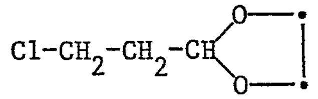

bzw. mit einem Organometallderivat, z.B. gemäss der obigen Deutschen Offenlegungsschrift 2 237 143, zu einer Verbindung der Formel

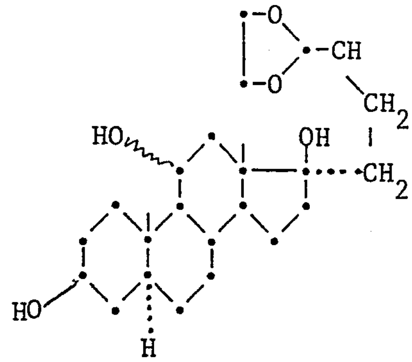

(XIV)

umgesetzt werden, welche dann nach der Methodik der Deutschen Offenlegungsschrift 2 237 143 in das gewünschte Spirolacton übergeführt werden kann. Wird das oben genannte 3,11α- oder 3,11β-Dihydroxy-5α-androstan-17-on in der gleichen Weise mit einem Chlorpropylalkohol-Derivat, wie einem reaktionsfähigen Ester, umgesetzt, so erhält man zu den Verbindungen der obigen Formel (XIV) analoge Verbindungen mit einem funktionell abgewandelten 3-Hydroxypropyl-Rest in 17α-Stellung, die man in die Spiroxane mit gesättigtem A-Ring, 3-Ketogruppe und 11β- oder 11α-Hydroxygruppe überführen kann.

Die Ausgangsstoffe der Formeln (III)-(XIII) sind neu und bilden ebenfalls einen Gegenstand der vorliegenden Erfindung. Unter solchen Verbindungen sind insbesondere auch Verbindungen gemäss der Formel II zu zählen, worin aber $R_2$ = O und $R_1$ = H ist, oder

$R_2$ = 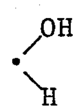 oder 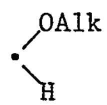 und $R_1$ = OH ist,

wobei Alk eine nieder-Alkylgruppe im oben definierten Sinne bedeutet. Solche Verbindungen stellen den 9α,11α-Epoxy-17-hydroxy-3-oxo-5α,17α-pregn-1-en-21-carbaldehyd oder 9α,11α-Epoxy-17β-hydroxy-17α-(3-oxo-propyl)-5α-androst-1-en-3-on, oder seine Halbacetale von Nieder-Alkanolen dar.

Neue Zwischenprodukte sind auch Verbindungen gemäss Formel (I) in denen X = 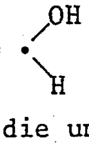 oder = 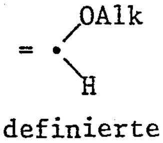 ist,

worbei Alk die unmittelbar oben definierte Bedeutung hat,

und welche das cyclische Halbacetal des vorhin genannten Aldehyds mit der 17β-Hydroxygruppe oder seine Niederalkyl-Aether darstellen.

Die Erfindung betrifft auch diejenigen Ausführungsformen der oben beschriebenen Verfahren, bei denen man von einer auf irgend einer Stufe als Zwischenprodukt erhältlichen Verbindung ausgeht und die fehlenden Schritte durchführt, oder bei denen ein Ausgangsstoff unter den Reaktionsbedingungen gebildet wird.

Die pharmakologisch verwendbaren Verbindungen der vorliegenden Erfindung der Formeln I und II können z.B. zur Herstellung von pharmazeutischen Präparaten, z.B. zur Behandlung von Hyperaldosteronismus verschiedenster Formen verwendet werden, welche eine wirksame Menge der Aktivsubstanz allein oder im Gemisch mit anorganischen oder organischen, festen oder flüssigen, pharmazeutisch verwendbaren Trägerstoffen enthalten, die sich insbesondere zu enteralen, z.B. oralen, und parenteralen Verabreichungen eignen. Vorzugsweise verwendet man Tabletten oder Gelatinkapseln, welche den Wirkstoff zusammen mit Verdünnungsmitteln, z.B. Laktose, Dextrose, Saccharose, Mannit, Sorbit, Cellulose und/oder Glycin, und Schmiermitteln, z.B. Kieselerde, Talk, Stearinsäure oder Salzen davon, wie Magnesium-oder Calciumstearat, und/oder Polyäthylenglykol, enthalten; Tabletten können ebenfalls Bindemittel, z.B. Magnesiumaluminiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, ferner auch Brausemischungen, Absorptionsmittel, Farbstoffe, Geschmackstoffe und Süssmittel enthalten, Injizierbare Präparate sind vorzugsweise isotonische wässrige Lösungen oder Suspensionen; Suppositorien sind in erster Linie Fettemulsionen oder -suspensionen. Die pharmakologischen Präparate können sterilisiert sein und/oder Hilfsstoffe, z.B. Konservier-, Stabilisier-, Netz- und/oder

Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0.1% bis etwa 75%, insbesondere von etwa 1% bis 50% des Aktivstoffes. Die empfohlene Tagesdosis für einen etwa 75 kg schweren Warmblüter beträgt 10-600 mg.

Gegenstand der vorliegenden Erfindung sind auch Zusammensetzungen enthaltend

A) mindestens eine Verbindung der Formeln (I) oder (II) gemäss der vorliegenden Erfindung und

B) mindestens ein in bezug auf die Elektrolytausscheidung unspezifisches Diuretikum,

gegebenenfalls zusammen mit pharmazeutischen Trägermaterialien.

Unter einem in bezug auf die Elektrolytausscheidung unspezifischen Diuretikum wird eines verstanden, welches sowohl Natrium- wie Kaliumionen in erhöhter Menge ausscheidet. Durch die Anwesenheit der Kaliumsparenden Komponente A) in den oben gekennzeichneten Zusammensetzungen wird die unerwünschte Kalium-ausscheidende Wirkung solcher Diuretika aufgehoben.

Als in bezug auf Elektrolytausscheidung unspezifische diuretische Komponente B kommen z.B. klassische Diuretika oder Gemische derselben in Betracht, die sowohl durch renale als auch durch extrarenale Wirkung auf die Gewebe die Diurese erhöhen, insbesondere Substanzen mit hemmender Wirkung auf die Rückresorption im Tubulus, wie Saluretika oder Aethacrinsäure und deren Analoge.

Besonders zu nennen sind Benzothiadiazin-Derivate, wie Thiazide und Hydrothiazide, ferner Benzolsulfonamide, Phenoxyessigsäuren, Benzofuran-2-carbonsäuren und 2,3-Dihydro-benzofuran-2-carbonsäuren.

Die Elektrolyt-unspezifische Komponente B kann aus einem einzelnen Wirkstoff oder einer zweckmässigen Kombination mehrerer Wirkstoffe bestehen, wobei die Wirkstoffe auch mehreren der genannten Stoffgruppen angehören können.

Besonders geeignete Thiazide sind z.B. 6-Chlor-7-sulfamyl-1,2,4-benzothiadiazin-1,1-dioxyd,6-Trifluormethyl-7-sulfamyl-1,2,4-benzothiadiazin-1,1-dioxyd und 2-Benzylthiomethyl-6-chlor-7-sulfamyl-1,2,4-benzothiadiazin-1,1-dioxyd.

Besonders geeignete Hydrothiazide sind z.B. 3-Aethyl-6-chlor-7-sulfamyl-3,4-dihydro-1,2,4-benzothiadiazin-1,1-dioxyd; 3-Trichlormethyl-6-chlor-7-sulfamyl-3,4-dihydro-1,2,4-benzothiadiazin-1,1-dioxyd; 3-Benzyl-6-trifluoromethyl-7-sulfamyl-3,4-dihydro-1,2,4-benzothiadiazin-1,1-dioxyd; 2-Methyl-3-(2,2,2-trifluoräthylthiomethyl)-6-chlor-7-sulfamyl-3,4-dihydro-1,2,4-benzothiadiazin-1,1-dioxid; 3-(2,2,2-Trifluoräthylthiomethyl)-6-chlor-7-sulfamyl-3,4-dihydro-1,2,4-benzothiadiazin-1,1-dioxyd; 3-(5-Norbornen-2-yl)-6-chlor-7-sulfamyl-3,4-dihydro-1,2,4-benzothiadiazin-1,1-dioxyd; 2-Methyl-3-chlormethyl-6-chlor-7-sulfamyl-3,4-dihydro-1,2,4-benzothiadiazin-1,1-dioxyd; 6-Chlor-7-sulfamyl-3,4-dihydro-1,2,4-benzothiadiazin-1,1-dioxyd; 3-Dichlormethyl-6-chlor-7-sulfamyl-3,4-dihydro-1,2,4-benzothiadiazin-1,1-dioxyd; 3-Cyclopentylmethyl-6-chlor-7-sulfamyl-3,4-dihydro-1,2,4-benzothiadiazin-1,1-dioxyd; 6-Trifluormethyl-7-sulfamyl-3,4-dihydro-1,2,4-benzothiadiazin-1,1-dioxyd und 3-Isobutyl-6-chlor-7-sulfamyl-3,4-dihydro-1,2,4-benzothiadiazin-1,1-dioxyd.

Besonders geeignete Benzolsulfonamide sind z.B. 2-Chlor-5-N-methylsulfonamido-benzol-sulfonamid; 2-Chlor-5-N,N-dimethylsulfonamido-benzol-sulfonamid; 2-Chlor-5-piperidinosulphonyl-benzol-sulfonamid;

2-Chlor-5-(N-carboxymethyl-N-methyl)-sulfonamido-benzol-sulfonamid; 2-Chlor-5-(N-furfuryl-sulfonamido)-benzol-sulfonamid; 2-Chlor-5-(N-tetrahydrofurfuryl-sulfonamido)-benzol-sulfonamid; 2-Chlor-5-[N-methyl-N-(2-methyl-4-oxo-tetrahydrofurfuryl)-sulfonamido]-benzol-sulfonamid; 4,5-Dichlorbenzol-1,3-disulfonamid; 4-Chlor-6-methylbenzol-1,3-disulfonamid; 4-Chlor-6-aminobenzol-1,3-disulfonamid; 2-Chlor-5-methylsulphonyl-benzol-sulfonamid; 2-Chlor-5-äthylsulphonyl-benzol-sulfonamid; 2-Chlor-5-n-butylsulphonyl-benzol-sulfonamid; 2-Methyl-5-äthylsulphonylbenzol-sulfonamid; 2-Methyl-5-methylsulphonyl-benzol-sulfonamid; 2-Methyl-5-n-butylsulphonyl-benzol-sulfonamid; 2-Chlor-4-(N,N-dibenzylamino)-5-carboxyl-benzol-sulfonamid; 2-Furfuryl-amino-4-chlor-5-N-(p-aminophenyl)-sulphamoyl-benzoesäure, 2-Furfuryl-amino-4-chlor-5-N-(O-aminophenyl)-sulphamoyl-benzoesäure und besonders 3-Sulfonamido-4-chlor-benzoesäure; 3-Sulfonamido-4-chlor-benzamid; 3-(N-methylsulphamoyl)-4-chlor-N-methylbenzamid; 1-Chlor-4-[N-methyl-N-(2-methyltetrahydrofurfuryl)-sulfamoyl]benzolsulfonamid; 1,3-Di-sulfamoyl-4-chlorbenzol; 2-Chlor-5-[3-hydroxy-1-oxo-isoindolyl-(3)]-benzol-sulfonamid; 2-Aethyl-4-oxo-6-sulfamoyl-7-chlor-1,2,3,4-tetra-hydro-chinazolin; 1-Oxo-2-cyclohexyl-5-chlor-6-sulfamoyl-1,2-dihydro-isoindol; 2-Chlor-5-[N-(2,6-dimethylpiperidino)-carbamoyl]-benzol-sulfonamid; 2-Chlor-4-furfurylamino-5-carboxyl-benzolsulfonamid und 2-Chlor-4-benzylamino-5-carboxyl-benzolsulfonamid.

Besonders geeignete Phenoxyessigsäuren sind z.B.
a) [2,3-Dimethyl-4-(2-methylen-butyryl)-phenoxy]-essigsäure, [2-Methyl-3-chlor-4-(2-methylen-butyryl)-phenoxy]-essigsäure, [4-(2-Methylenbutyryl)-1-naphthoxy]-essigsäure und vor allem [2,3-Dichlor-4-(2-methylen-butyryl)-phenoxy]-essigsäure;
b) 4-Thenoyl-2,3-dichlorphenoxyessigsäure, 4-(5-Methylthenoyl)-2,3-dichlorphenoxyessigsäure und 4-Furoyl-2,3-dichlorphenoxyessigsäure und
c) (1-Oxo-2-methyl-2-phenyl-6,7-dichlor-5-indanyloxy)-essigsäure (insbesondere als Razemat oder die laevo-Form), oder auch [1-Oxo-2-(4-chlorphenyl)-6,7-dichlor-5-indanyloxy]-essigsäure und [1-Oxo-2-(2-thienyl)-6,7-dichlor-5-indanyloxy]-essigsäure.

Besonders geeignete Benzofuran-2-carbonsäuren sind z.B. 5-(2-Methylen-butyryl)-6-methyl-benzofuran-2-carbonsäure, 5-(2-Methylen-butyryl)-6-methoxy-benzofuran-2-carbonsäure und 5-(2-methylen-propionyl)-6-methyl-benzofuran-2-carbonsäure.

Besonders geeignete 2,3-Dihydro-benzofuran-2-carbonsäuren sind z.B. 5-(2-Methylen-butyryl)-6-methyl-2,3-dihydro-benzofuran-2-carbonsäure; 5-(2-Methylen-butyryl)-6-fluor-2,3-dihydro-benzofuran-2-carbonsäure; 5-(2-Methylen-butyryl)-6-chlor-2,3-dihydro-benzofuran-2-carbonsäure; 5-(2-Methylenpropionyl)-6-methyl-2,3-dihydro-benzofuran-2-carbonsäure; 5-(2-Methylen-hexanoyl)-6-methyl-2,3-dihydro-benzofuran-2-carbonsäure; 5-(2-Methylen-valeryl)-6-methyl-2,3-dihydrobenzofuran-2-carbonsäure; 5-(2-Methylenpropionyl)-6-methyl-2,3-dihydro-benzofuran-2-carbonsäure; 5-(2-Aethyliden-butyryl)-6-methyl-2,3-dihydro-benzofuran-2-carbonsäure; 5-(2-Methylenbutyryl)-6-methyl-2,3-dihydro-benzofuran-2-carbonsäure-methylester; 5-(2-Methylenbutyryl)-6-methyl-2,3-dihydrobenzofuran-2-carbonsäureäthylester; 5-(2-Methylenbutyryl)-6-methyl-2,3-dihydro-benzofuran-2-carbonsäure-n-butylester; 5-(2-Methylenbutyryl)-6-methyl-2,3-dihydro-benzofuran-2-carbonsäure-2-hexylester; 5-(2-Methylenbutyryl)-6-methyl-2,3-dihydro-benzofuran-2-carbonsäure-n-decylester; 5-(2-Methylenbutyryl)-6-methyl-2,3-dihydro-benzofuran-2-carbonsäure-cyclopentylester; 5-(2-Methylenbutyryl)-6-methyl)-2,3-dihydrobenzofuran-2-carbonsäure-cyclohexylester; 5-(2-Methylenbutyryl)-6-methyl-2,3-dihydro-benzofuran-2-carbonsäure-benzylester; 5-(2-Methylenbutyryl)-7-methyl-2,3-dihydro-benzofuran-2-carbonsäure-methylester; 5-(2-Methylenbutyryl)-6-chlor-7-methyl-2,3-dihydro-benzofuran-2-carbonsäuremethylester; 5-(2-Methylenpropionyl)-6-methyl-2,3-dihydro-benzofuran-2-carbonsäuremethylester; 5-(2-Methylen-valeryl)-6-methyl-2,3-dihydro-benzofuran-2-carbonsäure-methylester; 5-(2-Methylen-3-methyl-butyryl)-6-methyl-2,3-dihydro-benzofuran-2-carbonsäure-methylester; und 5-(2-Methylenbutyryl)-6-fluor-2,3-dihydro-benzofuran-2-carbonsäure-methylester, und besonders 5-(2-Methylen-butyryl)-6,7-dimethyl-2,3-dihydro-benzofuran-2-carbonsäure; 5-(2-Methylen-3-methyl-butyryl)-6-methyl-2,3-dihydro-benzofuran-2-carbonsäure; 5-(2-Methylenbutyryl)-6-chlor-7-methyl-2,3-dihydro-benzofuran-2-carbonsäure.

Die genannten Diuretika können je nach Anzahl ihrer asymmetrischen Kohlenstoffatome in Form von Isomerengemischen, reinen Isomeren (Racematen) oder optischen Antipoden vorliegen. Vorzugsweise verwendet man sie jeweils in Form des besser wirksamen bzw. weniger toxischen Isomeren bzw. Antipoden.

Die genannten Diuretika mit basischen Gruppen können ferner in freier Form oder in Form ihrer nicht-toxischen Salze vorliegen. Als solche Salze kommen insbesondere Salze mit organischen oder anorganischen Säuren in Betracht, wie z.B.: Halogenwasserstoffsäuren, Schwefelsäuren, Phosphorsäuren, Salpetersäure, Perchlorsäure, aliphatische, alicyclische, aromatische oder heterocyclische Carbon- oder Sulfonsäuren, wie Ameisen-, Essig-, Propion-, Bernstein-, Glykol-, Milch-, Aepfel-, Wein-, Zitronen-, Ascorbin-, Malein-, Hydroxymalein- oder Brenztraubensäure; Phenylessig-, Benzoe-, p-Aminobenzoe-, Anthranil-, p-Hydroxybenzoe-, Salicyl- oder p-Aminosalicylsäure, Methansulfon-, Aethansulfon-, Hydroxyäthansulfon-, Aethylensulfonsäure; Halogenbenzolsulfon-, Toluolsulfon-, Naphthalinsulfonsäure oder Sulfanilsäure; Cyclohexyl-sulfaminsäure, Methionin, Tryptophan, Lysin oder Arginin.

Die genannten Diuretika mit sauren Gruppen können ebenfalls in freier Form oder in Form ihrer nicht-toxischen Salze vorliegen. Als solche Salze kommen insbesondere Salze mit Basen in Betracht, wie sie eingangs bei den Verbindungen der Formeln I und II genannt wurden. Aluminiumsalze, z.B. Salze aus zwei Mol Säure und einem Mol Aluminiumhydroxyd, sind ebenfalls geeignet, insbesondere wegen ihrer langsameren Resorption, Geruchlosigkeit und der geringen gastrointestinalen Störungen.

Die Erfindung betrifft auch die Herstellung der erfindungsgemässen Zusammensetzung und Arzneimittel, ferner die Anwendung der genannten Kombinationen der Wirkstoffe A und B, und zwar sowohl in Form der genannten Zusammensetzungen bzw. Arzneimittel wie auch durch eine

kombinierte aber getrennte Verabreichung beider Wirkstoffe, zur Behandlung von krankhaften Zuständen, die mit gestörter Ausscheidung von Harn, bzw. Harnbestandteilen, wie insbesondere von Wasser und Elektrolyten, einhergehen.

Besonders wertvoll sind pharmazeutische Zusammensetzungen und Arzneimittel, enthaltend die Aldosteron-antagonisierende Komponente A gemäss der Erfindung und als Komponente B 1-Oxo-3-(3-sulfamyl-4-chlor-phenyl)-3-hydroxyisoindolin, 6-Chloro-7-sulfamyl-3,4-dihydro-1,2,4-benzothiadiazin-1,1-dioxyd, 3-Cyclopentylmethyl-6-chloro-7-sulfamyl-3,4-dihydro-1,2,4-benzothiadiazin-1,1-dioxyd, 4-(2-Methylen-butyryl)-2,3-dichlor-phenoxyessigsäure, 4-Thenoyl-2,3-dichlor-phenoxyessigsäure, [1-Oxo-2-methyl-2-phenyl-6,7-dichlor-5-indanyloxy)-essigsäure, 2-Chlor-4-furfurylamino-5-carboxybenzolsulfonamid, 2-Phenoxy-2-butylamino-5-carboxybenzolsulfonamid oder 2-Phenoxy-3-[3-(1-pyrrolyl)-propyl]-5-carboxybenzolsulfonamid.

In den erfindungsgemässen pharmazeutischen Zusammensetzungen und Arzneimitteln beträgt das Verhältnis der Komponente A zur Komponente B, bezogen auf die jeweilige mittlere effektive Dosis, etwa 4:1 bis etwa 1:4, vorzugsweise etwa 3:2 bis etwa 2:3. Da die mittlere effektive Dosis jeder spezifischen Komponente ein bekannter, oder durch bekannte pharmakologische Testmethoden einfach feststellbarer Wert ist, ist dem Fachmann leicht möglich, innerhalb der obengenannten Grenzen ein geeignetes Verhältnis beider Komponenten jedem Patienten gemäss seinem spezifischen Leiden, allgemeinen Gesundheitszustand, individueller Ansprechbarkeit und Alter, sowie auch der Species, zu verordnen.
- Auch die Grösse der Dosierungseinheiten der erfindungsgemässen Arzneimittel hängt natürlich in erster Linie von der Wirksamkeit der jeweiligen Komponenten A bzw. B, wie sie vorzugsweise durch die Tagesdosis ausgedrückt ist, ab. Der Ausdruck "Dosierungseinheit" ist in dieser Beziehung angewendet zur Bezeichnung von einzelnen abgetrennten Portionen einheitlicher Zusammensetzung, welche für die medizinische Verabreichung geeignet sind und je einzeln eine spezifische Menge der erfindungsgemässen Wirkstoffe enthalten, die etwa 0,05 bis etwa 2,

vorzugsweise etwa 0,15 bis etwa 1 Tagesdosis entspricht.

So können beispielsweise die oben genannten besonders bevorzugten Präparate pro Dosiseinheit 15 bis 150 mg, insbesondere 20 bis 100 mg eines der genannten Verbindungen als Komponente A enthalten. Der Gehalt an Komponente B beträgt beispielsweise 10 - 100 mg, insbesondere 25 - 50 mg 2-Chlor-5-[3-hydroxy-1-oxo-isoindolyl-(3)]-benzolsulfonamid oder 4-(2-Methylenbutyryl)-2,3-dichlorphenoxyessigsäure, 5 - 50 mg, insbesondere 12 - 25 mg 6-Chloro-7-sulfamyl-3,4-dihydro-1,2,4-benzothiadiazin-1,1-dioxyd oder 2-Chlor-4-furfurylamino-5-carboxybenzolsulfonamid, 2 - 20 mg, insbesondere 5 - 10 mg 2-Phenoxy-3-[3-(1-pyrrolyl)-propyl]-5-carboxybenzolsulfonamid, 0,1 - 1,0 mg, insbesondere 0,25 - 0,5 mg 3-Cyclopentylmethyl-6-chloro-7-sulfamyl-3,4-dihydro-1,2,4-benzothiadiazin-1,1-dioxyd oder 2-Phenoxy-3-butylamino-5-carboxybenzolsulfonamid, 100 - 400 mg, insbesondere 200 mg 4-Thenoyl-2,3-dichlor-phenoxyessigsäure und 5 - 25 mg, insbesondere 10 mg racemische (1-Oxo-2-methyl-2-phenyl-6,7-dichlor-5-indanyloxy)-essigsäure, oder eine halbe Menge der laevo-Form dieser Säure.

Für die Oedembehandlung in einem mittelschweren Fall werden beispielsweise 1 - 3 Dosiseinheiten täglich genommen, die die Wirkstoffe in Gewichtsmengen enthalten, welche an der höheren Grenze der oben erwähnten besonders bevorzugten Dosierung liegen; ein mittelschwerer Fall der essentiellen Hypertonie wird z.B. mit 1-3 Dosiseinheiten behandelt, deren Wirkstoffgehalt an der unteren besonders bevorzugten Mengengrenze liegt.

In den folgenden Beispielen, welche die Erfindung weiter illustrieren, ohne sie dabei einzuschränken, sind die Temperaturen in Celsiusgraden angegeben.

Beispiel 1: 1,4 g (5α)-20-Spiroxa-1,9(11)-dien-3,21-dion werden in 28 ml Methylenchlorid gelöst, mit 1,4 g 90-%iger m-Chlorperbenzoesäure versetzt und zweieinhalb Stunden bei Raumtemperatur stehen gelassen. Nach Verdünnen mit Methylenchlorid wird je einmal mit einer ca. 10-%iger Kaliumjodid- und Natriumthiosulfatlösung und anschliessend mit eiskalter 1N-Natrolauge gewaschen. Die organische Phase wird nach dem Trocknen am Wasserstrahlvakuum eingedampft. Der kristalline Rückstand wird an 50-facher Gewichtsmenge Kieselgel chromatographiert. Mit einem Toluol-Aceton-(90:10)-Gemisch wird das reine 9α,11α-Epoxy-(5α)-20-spirox-1-en-3,21-dion eluiert, das nach einmaligem Umlösen aus Methylenchlorid-Aether einen Schmelzpunkt von 275-277° aufweist. Diese Verbindung weist im oben genannten Kagawa-Test eine $ED_{50}$ von 3-5 mg/kg p.o. auf. Den Ausgangsstoff kann man wie folgt herstellen:

Eine Lösung von 1,57 g 11α-Hydroxy-(5α)-20-spirox-1-en-3,21-dion in 30 ml Chloroform wird bei einer Innentemperatur von -50° innert 30 Sekunden mit 1,65 ml Piperidino-schwefeltrifluorid versetzt und während 30 Minuten bei einer Innentemperatur von -40° nachgerührt. Die Reaktionslösung wird mit 31,4 ml Wasser versetzt und 5 Minuten ohne Kühlung rühren gelassen. Nach weiterem Verdünnen mit Wasser und Chloroform wird die organische Phase je einmal mit eiskalter 1N-Natronlauge und eiskalter 2N-Salzsäure gewaschen, getrocknet und im Wasserstrahlvakuum eingedampft. Durch eine Kieselgel-Chromatographie (50-fache Gewichtsmenge) wird mit einem Toluol-Aethylacetat (92:8)-Gemisch das (5α)-20-Spiroxa-1,9(11)-dien-3,21-dion eluiert, das nach Umlösen aus Methylenchlorid/Aether bei 218-219° schmilzt.

Das 11α-Hydroxy-(5α)-20-spirox-1-en-3,21-dion kann man auf folgende Weise herstellen:

Eine auf ca. 0° abgekühlte Lösung von 89,6 g 3β,11α-Dihydroxy-(5α)-androstan-17-on-diacetat in 4 Liter abs. Tetrahydrofuran wird mit 11,2 g Lithium-Metall (in kleinen Stückchen) und 112 ml β-Chlor-propion-

aldehyd-äthylacetal versetzt und 3 Stunden unter Eiskühlung und weitere 15 Stunden bei Zimmertemperatur gerührt. Nach Aufgiessen auf ca. 12 Liter Eiswasser wird 2 mal mit Chloroform extrahiert, worauf man die organischen Phasen hintereinander 2 mal mit eiskalter, verdünnter Salzsäure und anschliessend mit Wasser wäscht, trocknet und am Wasserstrahlvakuum eindampft. Das so anfallende, gelbe Oel wird zwecks Nachacetylierung in je 500 ml Pyridin und Essigsäureanhydrid gelöst und über Nacht bei Zimmertemperatur stehen gelassen. Nach Aufgiessen auf ca. 6 Liter Eiswasser und 1-stündigem Rühren wird 2 mal mit Chloroform extrahiert. Die organischen Phasen werden je 2 mal mit verdünnter eiskalter Salzsäure, verdünnter eiskalter Natronlauge und Wasser gewaschen, getrocknet und im Wasserstrahlvakuum eingeengt. Die so erhaltene Lösung von ca. 500 ml wird durch Aluminiumoxid (neutral, Akt. Stufe II) filtriert und mit Chloroform nachgewaschen. Das durch Eindampfen der Chloroform-Eluate erhaltene Oel wird in 640 ml Methylenchlorid gelöst und bei einer Temperatur von 3-5° innerhalb von 2 Stunden mit 160 ml einer 8N Chrom-(VI)-schwefelsäurelösung versetzt und anschliessend 30 Minuten unter Eiskühlung und 2 Stunden bei Zimmertemperatur rühren gelassen. Das Reaktionsgemisch wird mit Methylenchlorid verdünnt und hintereinander mit eiskalter verdünnter Salzsäure, eiskalter verdünnter Natronlauge und Wasser gewaschen, getrocknet und im Wasserstrahlvakuum eingedampft. Das Rohprodukt wird an der 50-fachen Menge Kieselgel mit einem Toluol-Essigester-(90:10)-Gemisch chromatographiert.

7,4 g des so anfallenden 3β,11α-Dihydroxy-(5α)-20-spiroxan-21-on-diacetats, gelöst in 74 ml Methylalkohol und 29,6 ml Methylenchlorid werden mit 5,92 ml einer 3,15 N Salzsäure in Isopropylalkohol versetzt und 6 Stunden bei Zimmertemperatur gerührt und nach Verdünnen mit Eiswasser mit Chloroform extrahiert. Die organische Phase wird mit Wasser gewaschen, getrocknet und im Wasserstrahlvakuum eingedampft. Das amorphe Rohprodukt wird an Kieselgel chromatographiert, wobei mit einem Toluol-Aceton-(90:10)-Gemisch das

dünnschichtchromatographisch reine 3β,11α-Dihydroxy-(5a)-20-spiroxan-21-on-11-acetat erhalten wird.

7g dieses Monoacetats werden in 350 ml Aceton gelöst und unter Eiskühlung bei 5-8° mit 5,6 ml einer 8N Chrom-(VI)-schwefelsäurelösung versetzt und 30 Minuten unter Eiskühlung rühren gelassen. Nach Verdünnen mit Eiswasser wird mit Chloroform extrahiert, worauf man die organische Phase mit eiskalter verdünnter Natronlauge wäscht, trocknet und im Wasserstrahlvakuum eindampft.

8 g des so erhaltenen 11α-Hydroxy-(5α)-20-spiroxan-3,21-dion-acetats werden in 270 ml Methylalkohol gelöst, mit einer Lösung von 8 g Natriumhydroxid in 27 ml Wasser versetzt und während 3 Stunden unter Rückfluss gekocht. Nach Abkühlen auf Zimmertemperatur wird mit einer Lösung von 40 ml konz. Salzsäure in 27 ml Wasser versetzt und 30 Minuten stark gerührt. Nach Verdünnen mit Eiswasser wird mit Chloroform extrahiert, getrocknet und im Wasserstrahlvakuum eingedampft. Das amorphe Rohprodukt wird an der 50-fachen Menge Kieselgel mit einem Toluol-Aceton-(90:10)-Gemisch chromatographiert, wodurch das 11α-Hydroxy-(5α)-20-spiroxan-3,21-dion in reiner Form erhalten wird.

Eine auf 60° erwärmte Lösung von 5,82 g 11α-Hydroxy-(5α)-20-spiroxan-3,21-dion in 582 ml Tetrahydrofuran wird mit 7,27 g $CuBr_2$ versetzt und während 20 Minuten bei 60° gerührt. Das Reaktionsgemisch wird sofort auf 600 ml Eiswasser gegossen und 2 mal mit Aethylacetat extrahiert. Die organischen Phasen werden nacheinander mit 10-%iger Kaliumjodidlösung, 10-%iger Natriumthiosulfatlösung, eiskalter verdünnter Natronlauge, eiskalter verdünnter Salzsäure und mit gesättigter Kochsalzlösung gewaschen, getrocknet und im Wasserstrahlvakuum bei 40° eingedampft. Mit einem Toluol-Aceton-(90:10)-Gemisch wird das Rohprodukt an der 50-fachen Menge Kieselgel chromatographiert, wobei das 2α-Brom-11α-hydroxy-(5α)-20-spiroxan-3,21-dion rein, in amorpher Form erhalten wird.

6 g $2\alpha$-Brom-$11\alpha$-hydroxy-($5\alpha$)-20-spiroxan-3,21-dion werden in 60 ml Dimethylformamid gelöst, mit 3 g Lithiumcarbonat und 3 g Lithiumbromid versetzt und 90 Minuten am Rückfluss gekocht. Nach Abkühlen und Verdünnen mit Eiswasser wird 2 mal mit Aethylacetat extrahiert. Die organischen Phasen werden nacheinander mit eiskalter verdünnter Salzsäure, eiskalter verdünnter Natronlauge und gesättigter Kochsalzlösung gewaschen, getrocknet und im Wasserstrahlvakuum eingedampft. Mit einem Toluol-Aceton-(90:10)-Gemisch wird auf der 50-fachen Menge Kieselgel das $11\alpha$-Hydroxy-($5\alpha$)-20-spirox-1-en-3,21-dion eluiert, welches nach einmaligem Umlösen aus Methylenchlorid/Aether bei 279-281° schmilzt.

<u>Beispiel 2:</u> 3,2 g $9\alpha,11\alpha$-Epoxy-($5\alpha$)-20-spiroxan-3,21-dion werden in 95 ml Dioxan gelöst, mit 3,2 g 2,3-Dichlor-5,6-dicyano-1,4-benzochinon (DDQ) versetzt und in einem Glasbombenrohr während 16 Stunden bei 140° gerührt. Nach Eindampfen des Reaktionsgemisches wird der kristalline Rückstand auf 20 Dickschichtchromatographieplatten (à 1 Meter) präparativ getrennt (Laufmittel: Toluol-Aceton 75:25). Die im UV Licht 254 nm sichtbare Zone liefert nach dem Eluieren mit Aethylacetat, Eindampfen des Lösungsmittels und anschliessendem Umkristallisieren aus Methylenchlorid/Aether das $9\alpha,11\alpha$-Epoxy-($5\alpha$)-20-spirox-1-en-3,21-dion vom Schmelzpunkt 276-278°.

<u>Beispiel 3:</u> 1,9 g $9\alpha,11\alpha$-Epoxy-($5\alpha$)-20-spiroxan-3,21-dion werden in 76 ml Chlorbenzol gelöst, mit 1,9 g Benzolseleninsäureanhydrid versetzt und während 2 Stunden bei einer Aussentemperatur von 80° gerührt. Nach Abkühlen auf Zimmertemperatur werden 100 ml Wasser zugegeben und 30 Minuten gerührt. Die Reaktionslösung wird mit Chloroform extrahiert, worauf man die organische Phase nacheinander mit eiskalter verdünnter Salzsäure und eiskalter verdünnter Natronlauge wäscht, über Natriumsulfat trocknet und im Wasserstrahlvakuum eindampft. Das Rohprodukt wird an 240 g Kieselgel mit Methylenchlorid-Aceton 97:3 chromatographiert. Das anfallende $9\alpha,11\alpha$-Epoxy-($5\alpha$)-20-spirox-1-en-3,21-dion wird mit Methylenchlorid/Aether umgelöst und besitzt einen Schmelzpunkt von 276-278°.

Beispiel 4: Eine Lösung von 0,75 g 9α,11α-Epoxy-(5α)-20-spiroxan-3,21-dion in 16,8 ml Dioxan wird bei Zimmertemperatur innert 2 Minuten mit einer Bromlösung bestehend aus 2,29 ml Brom in 75 ml Dioxan versetzt und anschliessend eine weitere Minute bei Zimmertemperatur rühren gelassen. Die Reaktionslösung wird auf 75 ml Eiswasser, enthaltend 370 mg Natriumacetattrihydrat gegossen und mit Aethylacetat extrahiert. Man wäscht die organische Phase mit gesättigter Kochsalzlösung, trocknet sie über Natriumsulfat und dampft im Wasserstrahlvakuum bei max. 40° ein. Das Rohprodukt wird nach Lösen in 8,25 ml Dimethylformamid mit je 280 mg Lithiumcarbonat und Lithiumbromid versetzt und 1,5 Stunden unter Rückfluss gekocht. Das abgekühlte Gemisch wird mit 75 ml Eiswasser, enthaltend 1,90 ml konz. Salzsäure versetzt und 10 Minuten rühren gelassen. Nach 2-maliger Extraktion mit Aethylacetat werden die organischen Phasen mit gesättigter Natriumchloridlösung gewaschen, getrocknet und eingedampft. Mit einem Toluol-Aceton-(97:3)-Gemisch liefert das an 30-facher Gewichtsmenge Kieselgel chromatographierte Rohprodukt das 9α,11α-Epoxy-(5α)-20-spirox-1-en-3,21-dion, welches nach einmaligem Umlösen aus Methylenchlorid/Aether bei 276-278° schmilzt.

Beispiel 5: 2,14 g 9α,11α-Epoxy-(5α)-20-spirox-1-en-3,21-dion werden in 107 ml Tetrahydrofuran gelöst und bei Zimmertemperatur innert ca. 1 Minute mit 6,44 ml 0,84N wässriger Kaliumhydroxidlösung und innert ca. 2 Minuten mit 21 ml Wasser versetzt und während 18 Stunden bei 50° gerührt. Nach Abkühlen werden 70 ml Wasser zugegeben und das Tetrahydrofuran im Wasserstrahlvakuum abdestilliert. Nach Verdünnen mit 200 ml Wasser wird 3 mal mit je 200 ml Aether extrahiert, die wässrige Phase in einem $CO_2$/Aceton-Kühlbad eingefroren und im Hochvakuum lyophilisiert. Das Kalium-9α,11α-epoxy-17-hydroxy-3-oxo-5α,17α-pregn-1-en-21-carboxylat fällt in flockiger Form an.

Beispiel 6: Tabletten, enthaltend ca. 50 mg Wirkstoff, z.B. 9α,11α-Epoxy-(5α)-20-spirox-1-en-3-on oder 9α,11α-Epoxy-(5α)-20-spirox-1-en-3,21-dion werden wie folgt hergestellt:

Zusammensetzung für 1000 Tabletten

| | |
|---|---|
| Wirkstoff, feinst gemahlen | 50.0 g |
| Puderzucker (Saccharose) | 79.0 g |
| Arabisches Gummi | 4.75 g |
| Sorbit | 3.75 g |
| Talk | 2.5 g |
| Magnesiumstearat | 4.9 g |
| Mineralöl | 0.1 g |
| Carboxymethylcellulose (Na-Salz) | 5.0 g |

Herstellung:

Der Wirkstoff wird mit dem Puderzucker und dem arabischen Gummi vermischt, gesiebt und mittels einer etwa 35-prozentigen wässerigen Sorbit-Lösung granuliert. Das Granulat wird durch ein Sieb von 3 mm Maschenweite getrieben und im Wirbelschichttrockner bei 45° getrocknet, nochmals gesiebt und mit den übrigen Hilfsstoffen (Talk, Magnesiumstearat, Mineralöl und Carboxymethylcellulose-Natriumsalz) innig vermischt. Die Mischung wird in üblicher Weise zu Tabletten von 150 mg verpresst.

Beispiel 7: Gelatinekapseln enthaltend ca. 25 mg Wirkstoff, z.B. 9α,11α-Epoxy-(5α)-20-spirox-1-en-3,21-dion werden folgendermassen hergestellt:

Zusammensetzung für 1000 Kapseln

| | |
|---|---|
| Wirkstoff, feinst gemahlen | 25 g |
| Lactose, feinst gemahlen | 25 g |

Der Wirkstoff und die Lactose werden innig vermischt, verrieben und gesiebt, und das erhaltene Pulver in Portionen zu je 50 mg in Gelatinekapseln abgefüllt.

Beispiel 8: Tabletten, enthaltend ca. 100 mg Komponente A und ca. 25 mg Komponente B, werden folgendermassen hergestellt:

Zusammensetzung einer Tablette:

| | |
|---|---|
| Komponente A, mikronisiert | 100,0 mg |
| Komponente B, mikronisiert | 25,0 mg |
| Maisstärke | 50,0 mg |
| Kieselsäure, kolloidal | 5,0 mg |
| Gelatine | 5,0 mg |
| Cellulose mikrokristallin | 75,0 mg |
| Natriumcarboxymethylstärke | 20,0 mg |
| Magnesiumstearat | 1,5 mg |
| | 281,5 mg |

Herstellung von 100.000 Tabletten

10 kg Komponente A, mikronisiert, und 2,5 kg Komponente B, mikronisiert, 5,0 kg Maisstärke werden mit 0,5 kg kolloidaler Kieselsäure gemischt und mit einer Lösung von 0,5 kg Gelatine in 5,0 kg destilliertem Wasser (30°C) zu einer feuchten Masse verarbeitet. Diese wird durch ein Sieb von 3 mm Maschenweite getrieben und bei 45°C getrocknet (Wirbelschichttrockner). Das trockene Granulat wird durch ein Sieb von 0,8 mm Maschenweite gedrückt, mit einer vom voraus gesiebten Mischung von 7,5 kg mikrokristalliner Cellulose und 2,0 kg Natriumcarboxymethylstärke sowie 0,15 kg Magnesiumstearat gemischt und zu Tabletten von 281,5 mg Gewicht verpresst.

Als Komponente A wird 9α,11α-Epoxy-(5α)-20-spirox-1-en-3,21-dion, als Komponente B 6-Chlor-7-sulfamyl-3,4-dihydro-1,2,4-benzothiadiazin-1,1-dioxyd verwendet.

In analoger Weise kann man auch in den ensprechenden Mengen die folgenden Wirkstoffe einsetzen:

Als Komponente A: Das Kalium- oder Natriumsalz der 9$\alpha$,11$\alpha$-Epoxy-17-hydroxy-3-oxo-5$\alpha$,17$\alpha$-pregn-1-en-21-carbonsäure (100 mg);

als Komponente B: 2-Chlor-5-(3-hydroxy-1-oxo-isoindolyl-3)-benzolsulfonamid (50 mg) 4-(2-Methylenbutyryl)-2,3-dichlorphenoxy-essigsäure (50 mg), (1-Oxo-2-methyl-2-phenyl-6,7-dichlor-indanyl-5-oxy)-essigsäure (als Razemat 20 mg, als die laevo-Form 10 mg) oder 3-Cyclopentylmethyl-6-chlor-7-sulfamyl-3,4-dihydro-1,2,4-benzothiadiazin-1,1-dioxid (0,5 mg).

Patentansprüche (für alle Länder ausser AT)

1. Ein (5α)-20-Spirox-1-en-3-on der Formel

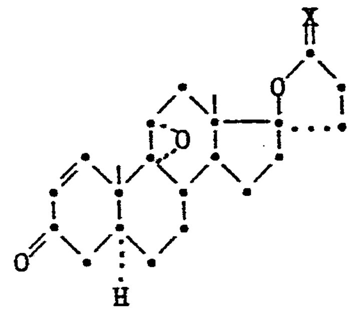

(I)

worin X = O oder $H_2$ ist, oder ein 17-Hydroxy-5α,17α-pregn-1-en-3-on der Formel

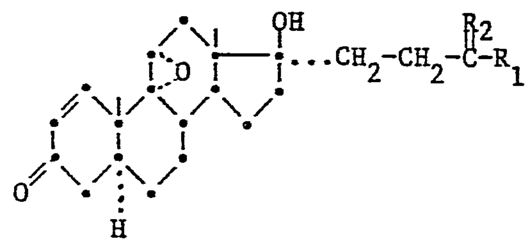

(II),

worin $R_2$ = O und $R_1$ = OH, OMe, OAlk, $NH_2$, $NHR_3$, $NR_3R_4$ oder $R_2$ = $H_2$ und $R_1$ = OH, OAlk, OAr, OAralk oder OAc ist,

wobei $R_3$, $R_4$ und Alk nieder-Alkyl, Me ein Metallatom bzw. -äquivalent oder das Kation einer organischen Base, Ar monocyclisches Aryl, Aralk monocyclisches Aryl-nieder-Alkyl, und Ac nieder-Alkanoyl, monocyclisches Aroyl, nieder-Alkylsulfonyl oder monocyclisches Arylsulfonyl bedeuten, wobei $R_3$ mit $R_4$ zusammen auch eine gegebenenfalls durch ein Heteroatom unterbrochene nieder-Alkylengruppe bedeuten kann.

2. Eine Verbindung der Formel II gemäss Anspruch 1, worin $R_2$ = O und $R_1$ = OH ist, oder ein Alkalimetallsalz davon.

3. Das 9α,11α-Epoxy-(5α)-20-spirox-1-en-3,21-dion.

4. Das 9α,11α-Epoxy-(5α)-20-spirox-1-en-3-on.

5. Die 9α,11α-Epoxy-17-hydroxy-3-oxo-5α,17α-pregn-1-en-21-carbonsäure oder ihr Natrium- oder Kaliumsalz.

6. Das 9α,11α-Epoxy-17-hydroxy-17α-(3-hydroxypropyl)-5α-androst-1-en-3-on.

7. Verfahren zur Herstellung einer Verbindung gemäss Anspruch 1, dadurch gekennzeichnet, dass man

a) eine Verbindung der Formel

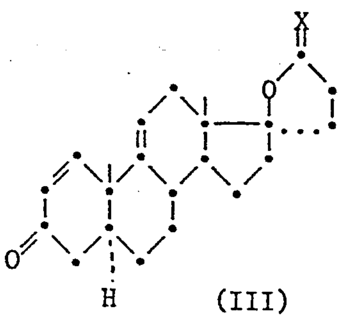

(III)

oder

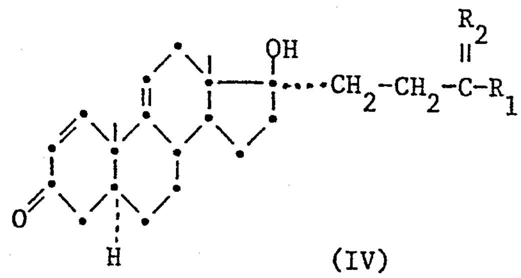

(IV)

worin X, $R_1$ und $R_2$ die oben angegebenen Bedeutungen haben, durch Behandeln mit einer Persäure zum entsprechenden 9α,11α-Epoxyd oxydiert, oder

b) in einer Verbindung der Formel

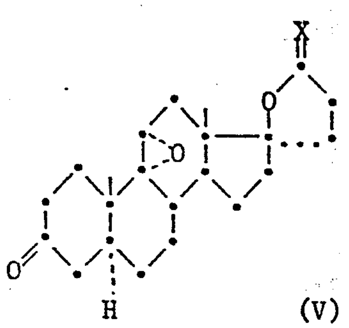

(V)

oder

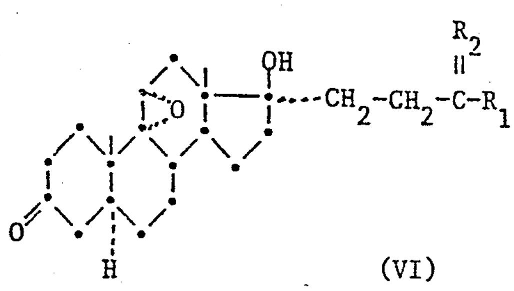

(VI)

worin X, $R_1$ und $R_2$ die oben angegebenen Bedeutungen haben, auf chemischem Wege eine Doppelbindung in 1,2-Stellung einführt, oder

c) in einer Verbindung der Formel

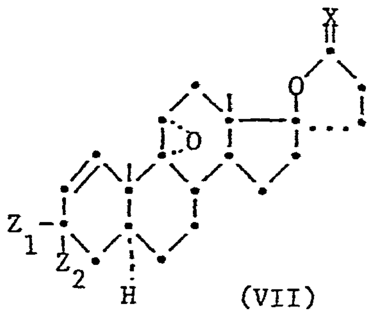

(VII)

oder

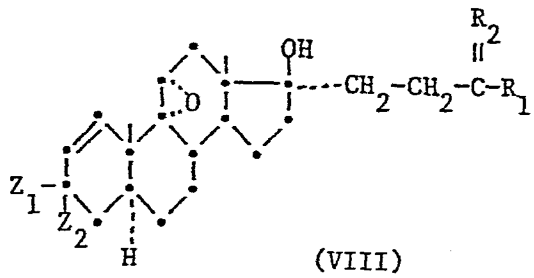

(VIII)

worin X, $R_1$ und $R_2$ die oben gegebenen Bedeutungen besitzen, und $Z_1$ und $Z_2$ eine in die Oxogruppe überführbare Gruppierung bedeutet, die Gruppe $Z_1$ und $Z_2$ in die 3-Oxogruppe überführt, oder

d) in einer Verbindung der Formel

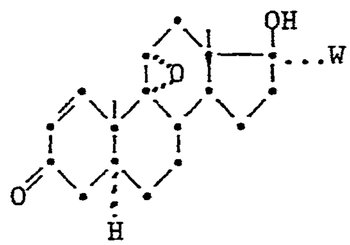

(IX),

worin W eine in 3'-Stellung durch eine reaktionsfähige funktionelle Gruppe oder eine sich davon ableitende abgewandelte Gruppe substituierte Propylgruppe, oder eine durch eine freie, veresterte oder amidierte Carboxylgruppe substituierte Aethylgruppe bedeutet, die Gruppe W unter Bildung eines Spiroäthers- oder Spirolactonringes mit der 17β-OH-Gruppe zur Reaktion bringt, oder

e) in einer Verbindung der Formel

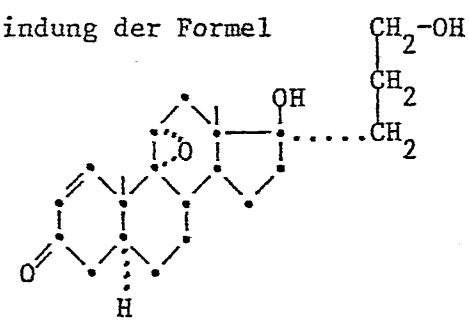

(X)

die Carbinolgruppe zur Carboxylgruppe gegebenenfalls unter Cyclisierung mit der 17β-Hydroxygruppe oxydiert, oder

f) eine Verbindung der Formel

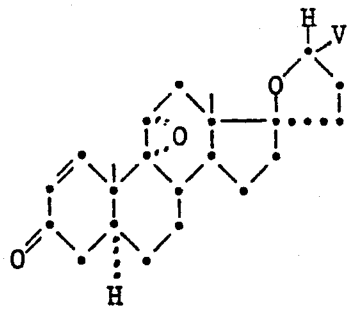

(XI)

in welcher V = H ist oder für eine freie, veresterte oder verätherte Hydroxy- oder Mercaptogruppe steht, zum entsprechenden Spirolacton oxydiert, oder

g) in einer Verbindung der Formel

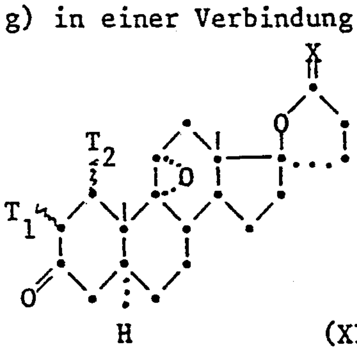

oder

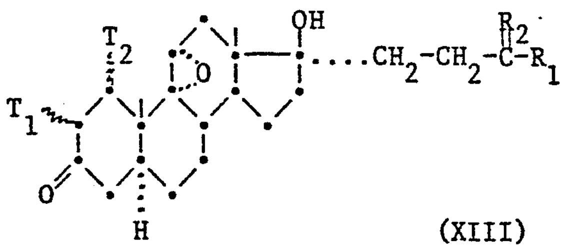

(XII) (XIII)

worin X, $R_1$ und $R_2$ die oben angegebenen Bedeutungen haben und eines der Substituenten $T_1$, $T_2$ eine Abgangsgruppe ist und der andere Wasserstoff bedeutet, die Abgangsgruppe unter Bildung der 1,2-Doppelbindung abspaltet, oder

h) in einer Verbindung der Formel (II), worin in der 17α-Seitenkette eine freie Hydroxy- oder Carboxylgruppe vorhanden ist, diese funktionell abwandelt, oder in einer Verbindung der Formel (II), worin eine funktionell abgewandelte Hydroxy- oder Carboxylgruppe vorhanden ist, diese in die entsprechende freie Gruppe überführt, oder

i) eine Verbindung der Formel (I), worin X = O ist, in eine entsprechende Carbonsäure gemäss Formel (II) überführt,

und, wenn erwünscht, die nach irgend einem der oben beschriebenen Verfahren erhaltene 9α,11α-Epoxy-17-hydroxy-3-oxo-5α,17α-pregn-1-en-21-carbonsäure in ihre Metallsalze oder Salze mit organischen Basen überführt oder nach einem der genannten Verfahren erhaltene Salze in die freie Säure überführt.

8. Pharmazeutische Präparate enthaltend eine oder mehrere Verbindungen gemäss einem der Ansprüche 1-6 zusammen mit mindestens einem pharmazeutischen Träger.

9. Eine Verbindung gemäss einem der Ansprüche 1-6 oder ein pharmazeutisches Präparat gemäss Anspruch 8 zur Verwendung als Aldosteron-antagonisierendes Mittel.

10. Eine pharmazeutische Zusammensetzung bestehend aus a) mindestens einer Verbindung der Formel (I) oder (II) gemäss Anspruch 1 und b) mindestens einem in bezug auf die Elektrolytausscheidung unspezifischem Diuretikum, zusammen mit mindestens einem pharmazeutischen Trägermaterial.

Patentansprüche (für AT)

1. Verfahren zur Herstellung eines (5α)-20-Spirox-1-en-3-ons der Formel

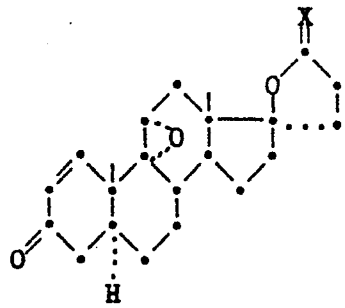

(I)

worin X = O oder $H_2$ ist, oder eines 17-Hydroxy-5α,17α-pregn-1-en-3-ons der Formel

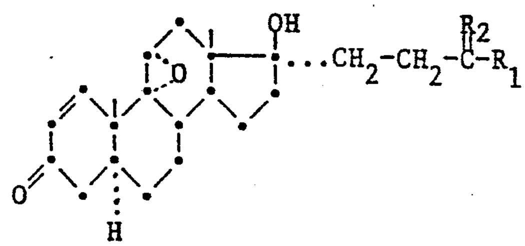

(II),

worin $R_2$ = O und $R_1$ = OH, OMe, OAlk, $NH_2$, $NHR_3$, $NR_3R_4$ oder $R_2$ = $H_2$ und $R_1$ = OH, OAlk, OAr, OAralk oder OAc ist,

wobei $R_3$, $R_4$ und Alk nieder-Alkyl, Me ein Metallatom bzw. -äquivalent oder das Kation einer organischen Base, Ar monocyclisches Aryl, Aralk monocyclisches Aryl-nieder-Alkyl, und Ac nieder-Alkanoyl, monocyclisches Aroyl, nieder-Alkylsulfonyl oder monocyclisches Arylsulfonyl bedeuten, wobei $R_3$ mit $R_4$ zusammen auch eine gegebenenfalls durch ein Heteroatom unterbrochene nieder-Alkylengruppe bedeuten kann, dadurch gekennzeichnet, dass man

a) eine Verbindung der Formel

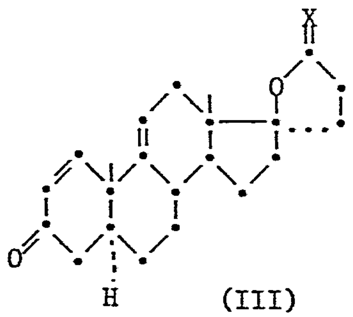

(III)

oder

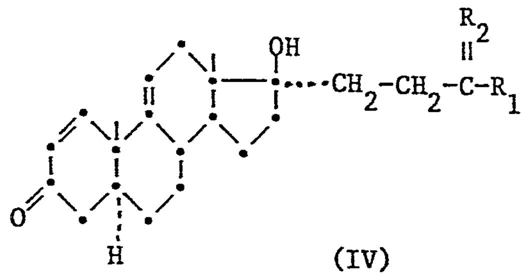

(IV)

worin X, $R_1$ und $R_2$ die oben angegebenen Bedeutungen haben, durch Behandeln mit einer Persäure zum entsprechenden 9α,11α-Epoxyd oxydiert, oder

b) in einer Verbindung der Formel

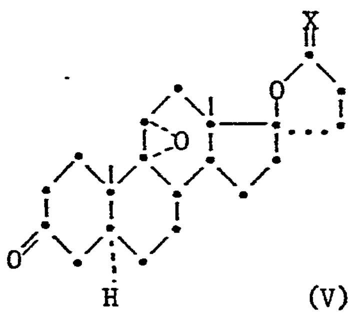

(V)

oder

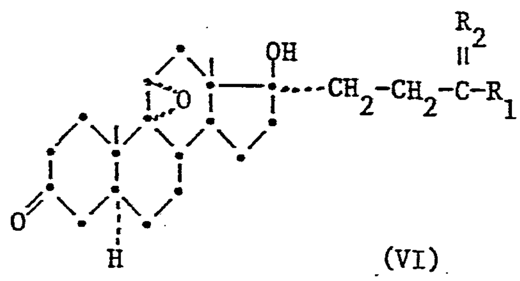

(VI)

worin X, $R_1$ und $R_2$ die oben angegebenen Bedeutungen haben, auf chemischem Wege eine Doppelbindung in 1,2-Stellung einführt, oder

c) in einer Verbindung der Formel

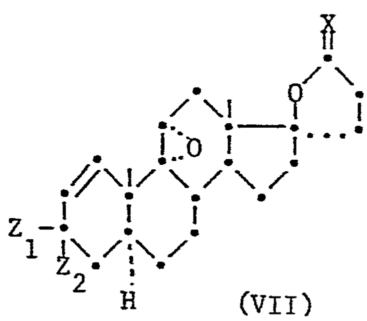

(VII)

oder

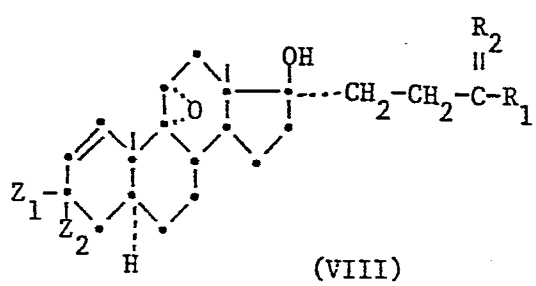

(VIII)

worin X, $R_1$ und $R_2$ die oben gegebenen Bedeutungen besitzen, und $Z_1$ und $Z_2$ eine in die Oxogruppe überführbare Gruppierung bedeutet, die Gruppe $Z_1$ und $Z_2$ in die 3-Oxogruppe überführt, oder

d) in einer Verbindung der Formel

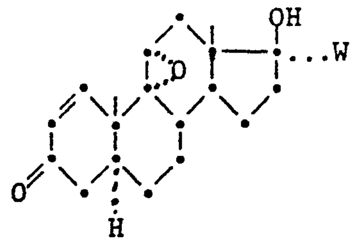

(IX),

worin W eine in 3'-Stellung durch eine reaktionsfähige funktionelle Gruppe oder eine sich davon ableitende abgewandelte Gruppe substituierte Propylgruppe, oder eine durch eine freie, veresterte oder amidierte Carboxylgruppe substituierte Aethylgruppe bedeutet, die Gruppe W unter Bildung eines Spiroäthers- oder Spirolactonringes mit der 17β-OH-Gruppe zur Reaktion bringt, oder

e) in einer Verbindung der Formel

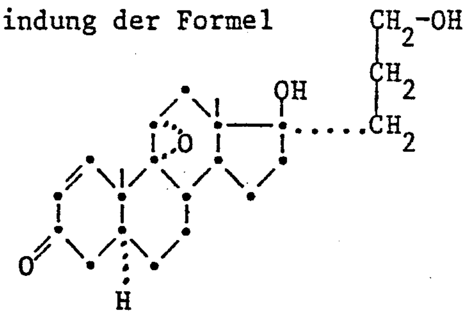

(X)

die Carbinolgruppe zur Carboxylgruppe gegebenenfalls unter Cyclisierung mit der 17β-Hydroxygruppe oxydiert, oder

f) eine Verbindung der Formel

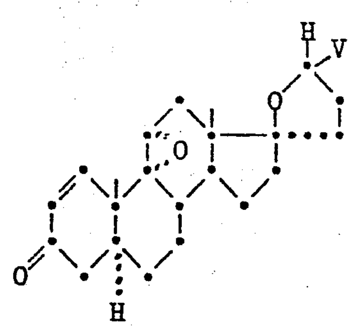

(XI)

in welcher V = H ist oder für eine freie, veresterte oder verätherte Hydroxy- oder Mercaptogruppe steht, zum entsprechenden Spirolacton oxydiert, oder

g) in einer Verbindung der Formel

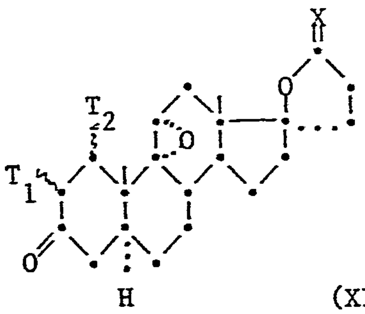 oder 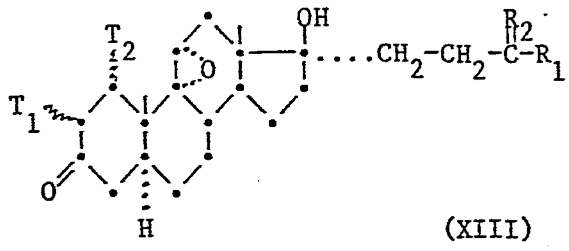

(XII) (XIII)

worin X, $R_1$ und $R_2$ die oben angegebenen Bedeutungen haben und eines der Substituenten $T_1$, $T_2$ eine Abgangsgruppe ist und der andere Wasserstoff bedeutet, die Abgangsgruppe unter Bildung der 1,2-Doppelbindung abspaltet, oder

h) in einer Verbindung der Formel (II), worin in der 17α-Seitenkette eine freie Hydroxy- oder Carboxylgruppe vorhanden ist, diese funktionell abwandelt, oder in einer Verbindung der Formel (II), worin eine funktionell abgewandelte Hydroxy- oder Carboxylgruppe vorhanden ist, diese in die entsprechende freie Gruppe überführt, oder

i) eine Verbindung der Formel (I), worin X = 0 ist, in eine entsprechende Carbonsäure gemäss Formel (II) überführt,

und, wenn erwünscht, die nach irgend einem der oben beschriebenen Verfahren erhaltene 9α,11α-Epoxy-17-hydroxy-3-oxo-5α,17α-pregn-1-en-21-carbonsäure in ihre Metallsalze oder Salze mit organischen Basen überführt oder nach einem der genannten Verfahren erhaltene Salze in die freie Säure überführt.

2. Verfahren gemäss Anspruch 1, dadurch gekennzeichnet, dass man eine Verbindung der Formel II, worin $R_2$ = 0 und $R_1$ = 0 ist, oder ein Alkalimetallsalz davon herstellt.

3. Verfahren gemäss Anspruch 1, dadurch gekennzeichnet, dass man das 9α,11α-Epoxy-(5α)-20-spirox-1-en-3,21-dion herstellt.

4. Verfahren gemäss Anspruch 1, dadurch gekennzeichnet, dass man das 9α,11α-Epoxy-(5α)-20-spirox-1-en-3-on herstellt.

5. Verfahren gemäss Anspruch 1, dadurch gekennzeichnet, dass man das 9α,11α-Epoxy-17-hydroxy-3-oxo-5α,17α-pregn-1-en-21-carbonsäure oder ihr Natrium- oder Kaliumsalz herstellt.

6. Verfahren gemäss Anspruch 1, dadurch gekennzeichnet, dass man das 9α,11α-Epoxy-17-hydroxy-17α-(3-hydroxypropyl)-5α-androst-1-en-3-on herstellt.

7. Verfahren zur Herstellung einer pharmazeutischen Zusammensetzung enthaltend eine Verbindung gemäss einem der Ansprüche 1-6, dadurch gekennzeichnet, dass man diese mit mindestens einem pharmazeutischen Trägermaterial auf nicht-chemischem Wege vermischt.

8. Verfahren zur Herstellung einer pharmazeutischen Zusammensetzung enthaltend a) mindestens eine Verbindung der Formeln (I) und (II) gemäss Anspruch 1 und b) mindestens ein in bezug auf die Elektrolytausscheidung unspezifisches Diuretikum, dadurch gekennzeichnet, dass man beide Wirkungskomponenten und mindestens ein pharmazeutisches Trägermaterial auf nicht-chemischem Wege vermischt.

9. Eine pharmazeutische Zusammensetzung hergestellt gemäss Anspruch 7.

10. Eine pharmazeutische Zusammensetzung hergestellt gemäss Anspruch 8.

Europäisches Patentamt

# EUROPÄISCHER RECHERCHENBERICHT

0122882
Nummer der Anmeldung

EP 84 81 0178

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | DE-A-2 303 216 (GD SEARLE)<br>* Ansprüche; Seiten 5-8 * | 1,8,9 | C 07 J 71/00<br>A 61 K 31/58<br>A 61 K 31/585//<br>C 07 J 21/00 |
| Y | US-A-3 095 412 (EDWARD A. BROWN)<br>* Whole document * | 1,8,9 | |
| D,A | SYNTHESIS, Nr. 11, November 1977, Seiten 773-774, Georg Thieme Verlag, Stuttgart, DE; E. MINCIONE et al.: "The regioselective alpha-bêta-dehydrogenation of ketosteroids by palladium(II)chloride"<br>* Verbindungen 1d und 2d * | 1,7 | |
| | | | RECHERCHIERTE SACHGEBIETE (Int. Cl. 3) |
| | | | C 07 J 71/00 |

Der vorliegende Recherchenbericht wurde für alle Patentansprüche erstellt.

| Recherchenort | Abschlußdatum der Recherche | Prüfer |
|---|---|---|
| DEN HAAG | 05-07-1984 | HENRY J.C. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82